(19) Europäisches Patentamt
European Patent Office
Office européen des brevets

(11) Publication number: **0 310 214 A2**

(12) **EUROPEAN PATENT APPLICATION**

(21) Application number: **88303571.9**

(22) Date of filing: **20.04.88**

(51) Int. Cl.[4]: **C07D 487/04 , A61K 31/415 , C07F 9/65 , //(C07D487/04, 231:00,231:00)**

Claims for the following Contracting States: ES + GR.

(30) Priority: **30.09.87 US 103488**
**29.10.87 US 114897**

(43) Date of publication of application:
**05.04.89 Bulletin 89/14**

(84) Designated Contracting States:
**AT BE CH DE ES FR GB GR IT LI LU NL SE**

(71) Applicant: **ELI LILLY AND COMPANY**
**Lilly Corporate Center**
**Indianapolis Indiana 46285(US)**

(72) Inventor: **Barnett, Charles Jackson**
**7540 North Pennsylvania**
**Indianapolis Indiana 46240(US)**
Inventor: **Holmes, Richard Elmer**
**4848 Laurel Circle**
**Indianapolis Indiana 46226(US)**
Inventor: **Jungheim, Louis Nickolaus**
**5706 N. Washington Boulevard**
**Indianapolis Indiana 46220(US)**
Inventor: **Sigmund, Sandra Kay**
**147 Trail View Drive Apartment Nr. 8**
**Carmel Indiana 46032(US)**
Inventor: **Ternansky, Robert John**
**1814 Deerbrook Drive**
**Noblesville Indiana 46060(US)**

(74) Representative: **Hudson, Christopher Mark et al**
**Erl Wood Manor**
**Windlesham Surrey GU20 6PH(GB)**

(54) **7-Substituted bicyclic pyrazolidinones.**

(57) 7-substituted bicyclic pyrazolidinone compounds as antimicrobials and the corresponding intermediates, are disclosed. The actual compounds are of the formula

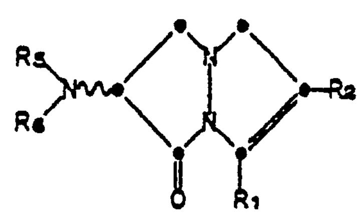

(I)

wherein:
$R_2$ is cyano;
a group of the formula

EP 0 310 214 A2

$$\overset{(O)_z}{\overset{\|}{-S-R_7}}$$

wherein Z is 0, 1 or 2 and $R_7$ is $C_1$ to $C_6$ alkyl, $C_2$ to $C_7$ alkenyl or $C_2$ to $C_7$ alkynyl;
a group of the formula

$$\overset{N-OR_8'}{\overset{\|}{-C-R_8}} \quad ,$$

wherein $R_8$ is hydrogen, $C_1$ to $C_6$ alkyl, $C_1$ to $C_6$ substituted alkyl, perfluoro $C_2$ to $C_4$ alkyl, trihalomethyl, $C_7$ to $C_{12}$ arylalkyl, $C_7$ to $C_{12}$ substituted arylalkyl, phenyl, substituted phenyl, amino, (monosubstituted)-amino, or (disubstituted)amino; and $R_8'$ is hydrogen, $C_1$ to $C_6$ alkyl, $C_2$ to $C_7$ alkenyl, $C_2$ to $C_7$ alkynyl, phenyl, substituted phenyl, $C_7$ to $C_{12}$ phenylalkyl, $C_7$ to $C_{12}$ substituted phenylalkyl;
or a group of the formula

$$\overset{O}{\overset{\|}{-NH-C-Nu}} \quad ,$$

wherein Nu is (monosubstituted)amino, (disubstituted)amino, $C_1$ to $C_6$ alkylthio, $C_2$ to $C_7$ alkenylthio, $C_1$ to $C_6$ substituted alkylthio, phenylalkylthio, $C_7$ to $C_1$ phenylalkylthio, or $C_7$ to $C_{12}$ substituted phenylalkylthio, $C_1$ to $C_6$ alkyl alcohol, $C_1$ to $C_6$ substituted alkyl alcohol, phenyl alcohol, substituted phenyl alcohol, $C_7$ to $C_{12}$ phenylalkyl alcohol, or $C_7$ to $C_{12}$ substituted phenylalkyl alcohol;
and $R_1$ is a group of the formula

$-COOR_{14}$

wherein $R_{14}$ is hydrogen, an organic or inorganic cation, a carboxy-protecting group or a non-toxic, metabolically-labile ester-forming group;
$R_5$ is hydrogen; and
$R_6$ is an acyl group of the formula

$-COR_{16}$

wherein $R_{16}$ is a group of the formula

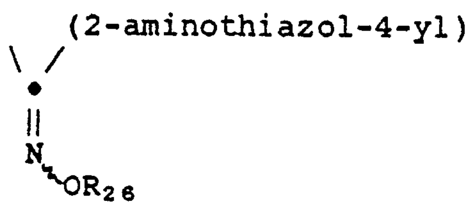

wherein $R_{26}$ is $C_2$ to $C_6$ alkenyl or $C_2$ to $C_7$ alkynyl; or a pharmaceutically-acceptable salt thereof.

# 7-SUBSTITUTED BICYCLIC PYRAZOLIDINONES

The present invention provides certain novel bicyclic pyrazolidinone antimicrobials which are useful in treating infectious diseases in man and other animals. The invention further provides intermediates useful in the preparation of these compounds and pharmaceutical formulations containing said bicyclic pyrazolidinones. In Sigmund, et al., European Patent Application No. 86303174.6 (Publication No. 0202046), bicyclic pyrazolidinone antimicrobials were disclosed.

Further aspects of the invention include pharmaceutical compositions and methods of treatment of gram-positive and gram-negative bacterial infections comprising the use of the above antimicrobial compounds.

The present invention provides compounds of the Formula I:

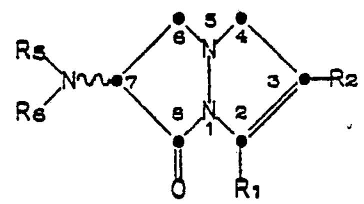

I

The ring system of the compound in Formula I is a 1,5-diazabicyclo[3.3.0]octa-2-ene ring, often referred to in this Specification as an "unsaturated bicyclic pyrazolidinone" or, more simply, a "bicyclic pyrazolidinone". The numbering system for the ring system is denoted in Formula I.

In the above Formula, the undulating lines connecting the nitrogen atom to position 7 of the ring system indicates that the stereochemistry at position 7 could be independently in the R or S configuration. Furthermore, the Formula represents compounds of the invention in various percentage mixtures of the possible enantiomeric and diastereomeric mixtures.

In the above Formula I:

$R_2$ is cyano;

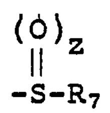

wherein Z is 0, 1 or 2 and $R_7$ is $C_1$ to $C_6$ alkyl, $C_2$ to $C_7$ alkenyl or $C_2$ to $C_7$ alkynyl; a group of the formula

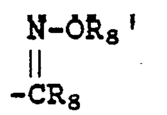

wherein $R_8$ is $C_1$ to $C_6$ alkyl, $C_1$ to $C_6$ substituted alkyl, perfluoro $C_2$ to $C_4$ alkyl, trihalomethyl, $C_7$ to $C_{12}$ phenylalkyl, $C_7$ to $C_{12}$ substituted phenylalkyl, phenyl, substituted phenyl, amino, (monosubstituted)amino or (disubstituted)amino; $R_8'$ is hydrogen, $C_1$ to $C_6$ alkyl, $C_2$ to $C_7$ alkenyl, $C_2$ to $C_7$ alkynyl, phenyl, substituted phenyl, $C_7$ to $C_{12}$ phenylalkyl, or $C_7$ to $C_{12}$ substituted phenylalkyl; or a group of the formula

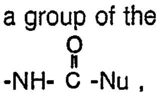

wherein Nu is (monosubstituted)amino, (disubstituted)amino, $C_1$ to $C_6$ alkylthio, $C_2$ to $C_7$ alkenylthio, $C_1$ to $C_6$ substituted alkylthio, phenylthio, substituted phenylthio, $C_7$ to $C_{12}$ phenylalkylthio, $C_7$ to $C_{12}$ substituted phenylalkylthio, or Nu is a $C_1$ to $C_6$ alkyl alcohol, $C_1$ to $C_6$ substituted alkyl alcohol, phenyl alcohol, substituted phenyl alcohol, $C_7$ to $C_{12}$ phenylalkyl alcohol, or $C_7$ to $C_{12}$ substituted phenyl alcohol residue; and $R_1$ is a group of the formula

$-COOR_{14}$

wherein $R_{14}$ is hydrogen, an organic or inorganic cation, a carboxy-protecting group, or a non-toxic, metabolically-labile ester-forming group;

$R_5$ is hydrogen and $R_6$ is an acyl group as defined below; or a pharmaceutically-acceptable salt thereof.

The protected amino, protected hydroxy and/or protected carboxy compounds represented by Formula I are intermediates to the compounds of Formula I where such groups are in the unprotected form. The unprotected form of the compounds of Formula I possess useful antimicrobial properties. The antimicrobial compounds of the invention can be used to inhibit the growth of microorganisms pathogenic to man and animals.

In one of its aspects, this invention provides a method for treatment of gram-positive and gram-negative bacterial infections, which comprises administering to an infected host a therapeutically effective amount of a compound of Formula I, wherein:

$R_{14}$ is hydrogen, an organic or inorganic cation, $C_1$ to $C_6$ alkyl, $C_1$ to $C_6$ substituted alkyl, $C_7$ to $C_{12}$ arylalkyl, $C_7$ to $C_{12}$ substituted arylalkyl, phenyl, substituted phenyl, or a non-toxic, metabolically-labile ester-forming group.

Yet another aspect of the invention is a pharmaceutical composition which comprises an anti-microbial compound useful in the above method and a suitable vehicle.

In the above Formula I, the term "$C_1$ to $C_6$ alkyl" denotes such radicals as methyl, ethyl, n-propyl, iso-propyl, n-butyl, sec-butyl, tert-butyl, amyl, tert-amyl, hexyl and the like. The preferred "$C_1$ to $C_6$ alkyl" group is methyl.

The term "$C_2$ to $C_7$ alkenyl" denotes such radicals as vinyl, allyl, 2-butenyl, 3-butenyl, 2-pentenyl, 3-pentenyl, 4-pentenyl, 2-hexenyl, 3-hexenyl, 4-hexenyl, 5-hexenyl, 2-heptenyl, 3-heptenyl, 4-heptenyl, 5-heptenyl, 6-heptenyl, as well as dienes and trienes of straight and branched chains. Allyl and 3-butene-1-yl are preferred embodiments.

The term "$C_2$ to $C_7$ alkynyl" denotes such radicals as ethynyl, propynyl, butynyl, pentynyl, hexynyl, heptynyl, as well as di- and tri-ynes.

The term "$C_1$ to $C_6$ substituted alkyl" denotes the above $C_1$ to $C_6$ alkyl groups that are substituted by one or two halogen, hydroxy, protected hydroxy, amino, protected amino, $C_1$ to $C_7$ acyloxy, nitro, carboxy, protected carboxy, carbamoyl, carbamoyloxy, cyano, methylsulfonylamino or $C_1$ to $C_4$ alkoxy groups. The substituted alkyl groups may be substituted once or twice with the same or with different substituents.

Examples of the above substituted alkyl groups include the cyanomethyl, nitromethyl, hydroxymethyl, trityloxymethyl, propionyloxymethyl, aminomethyl, carboxymethyl, allyloxycarbonylmethyl, allyloxycarbonylaminomethyl, carbamoyloxymethyl, methoxymethyl, ethoxy methyl, t-butoxymethyl, acetoxymethyl, chloromethyl, bromomethyl, iodomethyl, 6-hydroxyhexyl, 2,4-dichloro(n-butyl), 2-amino(iso-propyl), 2-carbamoyloxyethyl chloroethyl, bromoethyl, fluoroethyl, iodoethyl, chloropropyl, bromopropyl, fluoropropyl, iodopropyl and the like. A preferred group of examples within the above "$C_1$ to $C_6$ substituted alkyl" group includes the substituted methyl and substituted ethyl groups, in other words, a methyl or ethyl group substituted by the same substituents as the "$C_1$ to $C_6$ substituted alkyl" group. Examples of the substituted methyl or substituted ethyl groups includes groups such as hydroxymethyl, protected hydroxymethyl, (e.g., tetrahydropyranyloxymethyl), acetoxymethyl, carbamoyloxymethyl, chloromethyl, bromomethyl, iodomethyl, chloroethyl, bromoethyl, fluoroethyl, iodoethyl, chloropropyl, bromopropyl, fluoropropyl and iodopropyl.

The term "substituted phenyl" specifies a phenyl group substituted with one or two moieties chosen from the group consisting of halogen, hydroxy, protected hydroxy, cyano, nitro, $C_1$ to $C_6$ alkyl, $C_1$ to $C_4$ alkoxy, carboxy, protected carboxy, carboxymethyl, protected carboxymethyl, hydroxymethyl, protected hydroxymethyl, amino, protected amino, aminomethyl, protected aminomethyl, trifluoromethyl or N-(methylsulfonylamino).

Examples of the term "substituted phenyl" include a mono- or di(halo)phenyl group such as 4-chlorophenyl, 2,6-dichlorophenyl, 2,5-dichlorophenyl, 3,4-dichlorophenyl, 3-chlorophenyl, 3-bromophenyl, 4-bromophenyl, 3,4-dibromophenyl, 3-chloro-4-fluorophenyl, 2-fluorophenyl and the like; a mono- or di-(hydroxy)phenyl group such as 4-hydroxyphenyl, 3-hydroxyphenyl, 2,4-dihydroxyphenyl, the protected-hydroxy derivatives thereof and the like; a nitrophenyl group such as 3- or 4-nitrophenyl; a cyanophenyl group, for example, 4-cyanophenyl; a mono- or di(lower alkyl)phenyl group such as 4-methylphenyl, 2,4-dimethylphenyl, 2-methylphenyl, 4-(iso-propyl)phenyl, 4-ethylphenyl, 3-(n-propyl)phenyl and the like; a mono- or di(alkoxy)phenyl group, for example, 2,6-dimethoxyphenyl, 4-methoxyphenyl, 3-ethoxyphenyl, 4-(iso-propoxy)phenyl, 4-(t-butoxy)phenyl, 3-ethoxy-4-methoxyphenyl and the like; 3- or 4- trifluoromethylphenyl; a mono- or dicarboxyphenyl or (protected carboxy)phenyl group such as 4-carboxyphenyl or 2,4-di(protected carboxy)phenyl; a mono- or di(hydroxymethyl)phenyl or (protected hydroxymethyl)phenyl such as 3-(protected hydroxymethyl)phenyl or 3,4-di(hydroxymethyl)phenyl; a mono- or di(aminomethyl)phenyl or

(protected aminomethyl)phenyl such as 2-(aminomethyl)phenyl or 2,4-(protected aminomethyl)phenyl; or a mono- or di(N-(methylsulfonylamino))phenyl such as 3-(N-(methylsulfonylamino))phenyl. Also, the term "substituted phenyl" represents disubstituted phenyl groups wherein the substituents are different, for example, 3-methyl-4-hydroxyphenyl, 3-chloro-4-hydroxyphenyl, 2-methoxy-4-bromophenyl, 4-ethyl-2-hydroxyphenyl, 3-hydroxy-4-nitrophenyl, 2-hydroxy-4-chlorophenyl and the like. Preferred substituted phenyl groups include the 2- and 3-trifluoromethylphenyl, the 4-hydroxyphenyl, the 2-aminomethylphenyl and the 3-(N-(methylsulfonylamino))phenyl groups.

The terms "halo" and "halogen" refer to the fluoro, chloro, bromo or iodo groups.

The term "trihalomethyl" denotes trifluoromethyl, trichloromethyl, tribromomethyl or triiodomethyl.

The term "$C_7$ to $C_{12}$ phenylalkyl" denotes a $C_1$ to $C_6$ alkyl group substituted at any position by a phenyl ring. Examples of such a group include phenyl methyl (benzyl), 2-phenylethyl, 3-phenyl-(n-propyl), 4-phenylhexyl, 3-phenyl-(n-amyl), 3-phenyl-(sec-butyl), and the like. A preferred group is the benzyl group.

The term "$C_7$ to $C_{12}$ substituted phenylalkyl" denotes a $C_7$ to $C_{12}$ arylalkyl group substituted on the $C_1$ to $C_6$ alkyl portion with one or two groups chosen from halogen, hydroxy, protected hydroxy, amino, protected amino, $C_1$ to $C_7$ acyloxy, nitro, carboxy, protected carboxy, carbamoyl, carbamoyloxy, cyano, N-(methylsulfonylamino) or $C_1$ to $C_4$ alkoxy; and/or the phenyl group may be substituted with 1 or 2 groups chosen from halogen, hydroxy, protected hydroxy, nitro, $C_1$ to $C_6$ alkyl, $C_1$ to $C_4$ alkoxy, carboxy, protected carboxy, carboxymethyl, protected carboxymethyl, hydroxymethyl, protected hydroxymethyl, aminomethyl, protected aminomethyl, or a N-(methylsulfonylamino) group. As before, when either the $C_1$ to $C_6$ alkyl portion or the phenyl portion or both are disubstituted, the substituents can be the same or different.

Examples of the term "$C_7$ to $C_{12}$ substituted phenylalkyl" include groups such as 2-phenyl-1-chloroethyl, 2-(4-methoxyphenyl)ethyl, 2,6-dihydroxy-4-phenyl(n-hexyl), 5-cyano-3-methoxy-2-phenyl(n-pentyl), 3-(2,6-dimethylphenyl)n-propyl, 4-chloro-3-aminobenzyl, 6- (4-methoxyphenyl)-3-carboxy(n-hexyl), 5-(4-aminomethyl phenyl)-3-(aminomethyl)(n-pentyl), and the like.

As used above, the terms $C_1$ to $C_6$ alkyl alcohol, $C_1$ to $C_6$ substituted alkyl alcohol, phenyl alcohol (phenol), substituted phenyl alcohol, $C_7$ to $C_{12}$ phenyl alkyl alcohol, and $C_7$ to $C_{12}$ substituted phenylalkyl alcohol, all refer merely to the corresponding terms as defined above, each possessing one free hydroxy moiety. When used in conjunction with the group

$$-\overset{\displaystyle O}{\overset{\|}{C}}\text{-Nu},$$

the "alcohol residue" (Nu) refers to a compound wherein the oxygen of the alcohol is attached to the carbonyl group of

$$-\overset{\displaystyle O}{\overset{\|}{C}}\text{-Nu}.$$

The term "(monosubstituted) amino" refers to an amino group with one substituent chosen from the group consisting of phenyl, substituted phenyl, $C_1$ to $C_6$ alkyl, and $C_7$ to $C_{12}$ arylalkyl, wherein the latter three substituent terms are as defined above.

The term "(disubstituted)amino" refers to amino groups with two substituents chosen from the group consisting of phenyl, substituted phenyl, $C_1$ to $C_6$ alkyl, and $C_7$ to $C_{12}$ arylalkyl wherein the latter three substituent terms are as described above. The two substituents can be the same or different.

The term "organic or inorganic cation" refers to counter-ions for the carboxylate anion of a carboxylate salt. The counter-ions are chosen from the alkali and alkaline earth metals, (such as lithium, sodium, potassium, barium and calcium); ammonium; and the organic cations (such as dibenzylammonium, benzylammonium, 2-hydroxyethylammonium, bis(2-hydroxyethyl)ammonium, phenylethylbenzylammonium, dibenzylethylenediammonium, and like cations). Other cations encompassed by the above term include the protonated form of procaine, quinine and N-methylglucosamine, and the protonated forms of basic amino acids such as glycine, ornithine, histidine, phenylglycine, lysine and arginine. Furthermore, any zwitterionic form of the instant compounds formed by a carboxylic acid and an amino group is referred to by this term. For example, a cation for a carboxylate anion will exist when $R_2$ or $R_1$ is substituted with a (quaternary ammonium)methyl group. A preferred cation for the carboxylate anion is the sodium cation.

The term "pharmaceutically-acceptable salt" encompasses those salts that form with the carboxylate anions and includes salts formed with the organic and inorganic cations discussed above. Furthermore, the term includes salts that form by standard acid-base reactions with basic groups (such as amino groups) and organic or inorganic acids. Such acids include hydrochloric, sulfuric, phosphoric, acetic, succinic, citric, lactic, maleic, fumaric, palmitic, cholic, pamoic, mucic, D-glutamic, d-camphoric, glutaric, phthalic, tartaric, lauric, stearic, salicyclic, methanesulfonic, benzenesulfonic, sorbic, picric, benzoic, cinnamic, and like acids.

The compounds of Formula I may also exist as solvates and hydrates. Thus, these compounds may crystallize with, for example, waters of hydration, or one, a number of, or any fraction thereof of molecules

of the mother liquor solvent. The solvates and hydrates of such compounds are included within the scope of this invention.

The term "carboxy-protecting group" as used in the specification refers to one of the ester derivatives of the carboxylic acid group commonly employed to block or protect the carboxylic acid group while reactions are carried out on other functional groups on the compound. Examples of such carboxylic acid protecting groups include 4-nitrobenzyl, 4-methoxybenzyl, 3,4-dimethoxybenzyl, 2,4-dimethoxybenzyl, 2,4,6-trimethoxybenzyl, 2,4,6-trimethylbenzyl, pentamethylbenzyl, 3,4-methylenedioxybenzyl, benzhydryl, 4,4′-dimethoxybenzhydryl, 2,2′,4,4′-tetramethoxybenzhydryl, t-butyl, t-amyl, trityl, 4-methoxytrityl, 4,4′-dimethoxytrityl, 4,4′,4″-trimethoxytrityl, 2-phenylprop-2-yl, trimethylsilyl, t-butyldimethylsilyl, phenacyl, 2,2,2-trichloroethyl, β-(trimethylsilyl)ethyl, β-(di(n-butyl)methylsilyl)ethyl, p-toluenesulfonylethyl, 4-nitrobenzylsulfonylethyl, allyl, cinnamyl, 1-(trimethylsilylmethyl)prop-1-en-3-yl, and like moieties. The species of carboxy-protecting group employed is not critical so long as the derivatized carboxylic acid is stable to the condition of subsequent reaction(s) on other positions of the bicyclic pyrazolidinone molecule and can be removed at the appropriate point without disrupting the remainder of the molecule. In particular, it is important not to subject the carboxy-protected bicyclic pyrazolidinone molecule to strong nucleophilic bases or reductive conditions employing highly activated metal catalysts such as Raney nickel. (Such harsh removal conditions are also to be avoided when removing amino-protecting groups and hydroxy-protecting groups, discussed below.) A preferred carboxylic acid protecting group is the allyl group. Similar carboxy-protecting groups used in the cephalosporin, penicillin and peptide arts can also be used to protect a carboxy group substituents of the bicyclic pyrazolidinones. Further examples of these groups are found in E. Haslam, "Protective Groups in Organic Chemistry", J.G.W. McOmie, Ed., Plenum Press, New York, N.Y., 1973, Chapter 5, and T.W. Greene, "Protective Groups in Organic Synthesis", John Wiley and Sons, New York, N.Y., 1981, Chapter 5. A related term is "protected carboxy", which refers to a carboxy group substituted with one of the above carboxy-protecting groups.

The term "hydroxy-protecting group" refers to readily cleavable groups bonded to hydroxyl groups, such as the tetrahydropyranyl, 2-methoxyprop-2-yl, 1-ethoxyeth-1-yl, methoxymethyl, β-methoxyethoxymethyl, methylthiomethyl, t-butyl, t-amyl, trityl, 4-methoxytrityl, 4,4′-dimethoxytrityl, 4,4′,4″-trimethoxytrityl, benzyl, allyl, trimethylsilyl, (t-butyl)dimethylsilyl and 2,2,2-trichloroethoxycarbonyl groups and the like.

The species of hydroxy-protecting groups is not critical so long as the derivatized hydroxyl group is stable to the conditions of subsequent reaction(s) and can be removed at the appropriate point without disrupting the remainder of the bicyclic pyrazolidinone molecule.

Further examples of hydroxy-protecting groups are described by C.B. Reese and E. Haslam, "Protective Groups in Organic Chemistry", J.G.W. McOmie, Ed., Plenum Press, New York, N.Y., 1973, Chapters 3 and 4, respectively, and T.W. Greene, "Protective Groups in Organic Synthesis", John Wiley and Sons, New York, N.Y., 1981, Chapters 2 and 3. Some preferred hydroxy-protecting groups are the trityl group and the tetrahydropyranyl group. The related term "protected hydroxy" denotes a hydroxy group bonded to one of the above hydroxy-protecting groups.

The term "amino-protecting group" as used in the specification refers to substituents of the amino group commonly employed to block or protect the amino functionality while reacting other functional groups on the compound. Examples of such amino-protecting groups include the formyl group, the trityl group, the phthalimido group, the trichloroacetyl group, the chloroacetyl, bromoacetyl and iodoacetyl groups, urethane-type blocking groups such as benzyloxycarbonyl, 4-phenylbenzyloxycarbonyl, 2-methylbenzyloxycarbonyl, 4-methoxybenzyloxycarbonyl, 4-fluorobenzyloxycarbonyl, 4-chlorobenzyloxycarbonyl, 3-chlorobenzyloxycarbonyl, 2-chlorobenzyloxycarbonyl, 2,4-dichlorobenzyloxycarbonyl, 4-bromobenzyloxycarbonyl, 3-bromobenzyloxycarbonyl, 4-nitrobenzyloxycarbonyl, 4-cyanobenzyloxycarbonyl, 2-(4-xenyl)iso-propoxycarbonyl, 1,1-diphenyleth-1-yloxycarbonyl, 1,1-diphenylprop-1-yloxycarbonyl, 2-phenylprop-2-yloxycarbonyl, 2-(p-toluyl)-prop-2-yloxycarbonyl, cyclopentanyloxycarbonyl, 1-methylcyclopentanyloxycarbonyl, cyclohexanyloxycarbonyl, 1-methylcyclohexanyloxycarbonyl, 2-methylcyclohexanyloxycarbonyl, 2-(4-toluylsulfonyl)-ethoxycarbonyl, 2- (methylsulfonyl)ethoxycarbonyl, 2-(triphenylphosphino)ethoxycarbonyl, 9-fluorenylmethoxycarbonyl ("FMOC"), 2-(trimethylsilyl)ethoxycarbonyl, allyloxycarbonyl, 1-(trimethylsilylmethyl)prop-1-enyloxycarbonyl, 5-benzisoxalylmethoxycarbonyl, 4-acetoxybenzyloxycarbonyl, 2,2,2-trichloroethoxycarbonyl, 2-ethynyl-2-propoxycarbonyl, cyclopropylmethoxycarbonyl, 4-(decyloxy)-benzyloxycarbonyl, isobornyloxycarbonyl, 1-piperidyloxycarbonyl and the like; the benzoylmethylsulfonyl group, the 2-(nitro)phenylsulfenyl group, the diphenylphosphine oxide group and like amino-protecting groups. The species of amino-protecting group employed is not critical so long as the derivatized amino group is stable to the condition of subsequent reaction(s) on other positions of the bicyclic pyrazolidinone molecule and can be removed at the appropriate point without disrupting the remainder of the molecule.

Preferred amino-protecting groups are the allyloxycarbonyl, the t-butoxycarbonyl, and the trityl groups. Similar amino-protecting groups used in the cephalosporin, penicillin and peptide art are also embraced by the above terms. Further examples of groups referred to by the above terms are described by J.W. Barton, "Protective Groups In Organic Chemistry", J.G.W. McOmie, Ed., Plenum Press, New York, N.Y., 1973, Chapter 2, and T.W. Greene, "Protective Groups in Organic Synthesis", John Wiley and Sons, New York, N.Y., 1981, Chapter 7. The related term "protected amino" defines an amino group substituted with an amino-protecting group discussed above.

The term "non-toxic, metabolically-labile ester-forming group" refers to those biologically active ester forms which induce increased blood levels and prolong the efficacy of the corresponding non-esterified forms of the compounds. Such ester groups include the lower alkoxymethyl groups, for example, methoxymethyl, ethoxymethyl, iso-propoxymethyl and the like; the α-($C_1$ to $C_4$)alkoxyethyl groups, for example methoxyethyl, ethoxyethyl, propoxyethyl, iso-propoxyethyl, and the like; the 2-oxo-1,3-dioxolen-4-ylmethyl groups, such as 5-methyl-2-oxo-1,3-dioxolen-4-ylmethyl, 5-phenyl-2-oxo-1,3-dioxolen-4-ylmethyl, and the like; the $C_1$ to $C_3$ alkylthiomethyl groups, for example methylthiomethyl, ethylthiomethyl, iso-propylthiomethyl, and the like; the acyloxymethyl groups, for example pivaloyloxymethyl, pivaloyloxyethyl, α-acetoxymethyl, and the like; the ethoxycarbonyl-1-methyl group; the α-acyloxy-α-substituted methyl groups, for example α-acetoxyethyl; the 3-phthalidyl or 5,6-dimethylphthalidyl groups; the 1-($C_1$ to $C_4$ alkyloxycarbonyloxy)ethyl groups such as the 1-(ethoxycarbonyloxy)ethyl group; and the 1-($C_1$ to $C_4$ alkylaminocarbonyloxy)ethyl groups such as the 1-(methylaminocarbonyloxy)ethyl group.

In Formula (I) above, $R_5$ is hydrogen and the $R_6$ is an acyl group of the formula

$-COR_{16}$

wherein $R_{16}$ is:
a group of the formula

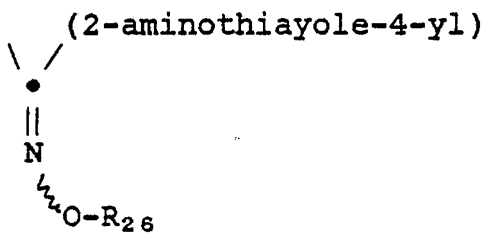

wherein $R_{26}$ is $C_2$ to $C_7$ alkenyl or $C_2$ to $C_7$ alkynyl.

Preferred examples of the acyl groups represented by $COR_{16}$ include:
2-(2-aminothiazol-4-yl)-2-(Z)-allyloximinoacetyl,
2-(2-aminothiazol-4-yl)-2-(Z)-vinyloximinoacetyl,
2-(2-aminothiazol-4-yl)-2-(Z)-(2-buten-1-yl)oximinoacetyl,
2-(2-aminothiazol-4-yl)-2-(Z)-(3-butene-1-yl)oximinoacetyl,
2-(2-aminothiazol-4-yl)-2-(Z)-(2-propyne-1-yl)oximinoacetyl,
and the protected amino, protected carboxy, amine salt and carboxylate salt derivatives thereof.

A further preferred $-COR_{16}$ acyl group is 2-(2-aminothiazol-4-yl)-2-(Z)-(3-butene-1-yl)oximinoacetyl.

Among the groups above which comprise many of the possibilities for the group

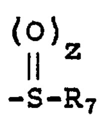 ,

the most preferred is methylsulfonyl.

In the above Formula I $R_{14}$ is a carboxyl group of the formula

$-COOR_{14}$

Examples include groups when $R_{14}$ is: $C_1$ to $C_6$ alkyl, such as ethoxycarbonyl, n-propoxycarbonyl, sec-butoxycarbonyl, t-amyloxycarbonyl and the like; $C_1$ to $C_6$ substituted alkyl, such as (3-cyanopropyloxy)-carbonyl, 4,5-dichloroamyloxycarbonyl, 2-carboxy-1-nitroethoxycarbonyl, and the like; phenyl (the phenoxycarbonyl group), substituted phenyl, for example, 4-methoxyphenoxycarbonyl, 2,4-dimethylphenoxycarbonyl, 3-nitrophenoxycarbonyl, 4-trifluoromethylphenoxycarbonyl, 2,4-di(methoxycarbonyl)phenoxycarbonyl, 2-(aminomethyl)phenoxycarbonyl, 3-hydroxy-4-nitrophenoxycarbonyl, and the like; $C_7$ to $C_{12}$ arylalkyl, such benzyloxycarbonyl, 2-phenylethoxycarbonyl, phenyl(t-butoxy)carbonyl, 3-phenylamyloxycarbonyl and the like; trihalomethyl, such as trifluoromethoxycarbonyl, trichloromethoxycarbonyl, tribromomethoxycarbonyl or triiodomethoxycarbonyl; or $C_7$ to $C_{12}$ substituted arylalkyl, such as 3-(3,4-diiodophenyl)propoxycarbonyl, 1-(3-chloro-4-fluorophenyl)ethoxycarbonyl, 6-(4-cyanophenyl)hexyloxycarbonyl, 3-phenyl-1-chloro(sec-butoxy)-carbonyl, 2-phenyl-2-hydroxyethoxycarbonyl, 5-phenyl-2-hydroxyamyloxycarbonyl, 2-(3-nitrophenyl)-3-ethoxypropoxycarbonyl, 5,6-dihydroxy-2-(4-ethyl-2-hydroxyphenyl)hexyloxycarbonyl, and 5-carbamoyl-3-nitro-(2,4-di methoxyphenyl)amyloxycarbonyl, and the like.

Further examples of the above $-COOR_{14}$ group are when $R_{14}$ is: an organic or inorganic cation, such as ammonium carboxylate, procaine carboxylate, (phenylethylbenzylammonium) carboxylate, phenylglycine carboxylate, lysine carboxylate, lithium carboxylate, potassium carboxylate, sodium carboxylate and the like; a carboxy-protecting group, such as allyl carboxylate, p-methoxybenzyl carboxylate, di-(4-methoxy)-benzhydryl carboxylate, benzhydryl carboxylate, 2,2,2-trichloroethyl carboxylate, trimethylsilyl carboxylate, (t-butyl)dimethylsilyl carboxylate, β-(trimethylsilyl)ethyl carboxylate, trityl carboxylate, 4,4′,4″-trimethoxytrityl carboxylate, p-toluenesulfonylethyl carboxylate, and the like; a non-toxic, metabolically-labile ester-forming group, such as methoxymethyl carboxylate, 5-methyl-2-oxo-1,3-dioxolen-4-ylmethyl carboxylate, ethylthiomethyl carboxylate, pivaloyloxymethyl carboxylate, 3-phthalidyl carboxylate, 1-(ethoxycarbonyloxy)ethyl carboxylate, 1-(methylaminocarbonyloxy)ethyl carboxylate, and the like.

A preferred group of examples of the carboxy group $-COOR_{14}$ is when $R_{14}$ is a carboxy-protecting group, hydrogen or an organic or inorganic cation. An especially preferred group of examples of the above carboxy group is when $R_{14}$ is benzyl, hydrogen, allyl, t-butyl, 4-nitrobenzyl, or sodium.

Above all, a preferred class of compounds within those defined by Formula I have the 7-(S) configuration represented by the formula:

A preferred group of 7-(S) oximino acyl compounds has $R_1$ as a group of the formula

$-COOR_{14}$,

$R_2$ as either cyano or a group of the formula

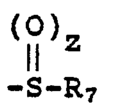
;

or a pharmaceutically-acceptable salt thereof.

Many of the bicyclic pyrazolidinones of Formula I may be prepared by the general methodology of Sigmund et al., European Patent Application No. 86303174.6 (Publication No. 0202046), incorporated herein by reference.

One synthetic route to 7-substituted bicyclic pyrazolidinones centers around the cyclization of a 1,2-(disubstituted)diazolidinone via a phosphorane reagent. This route is diagramed below in Scheme 1:

## Scheme 1

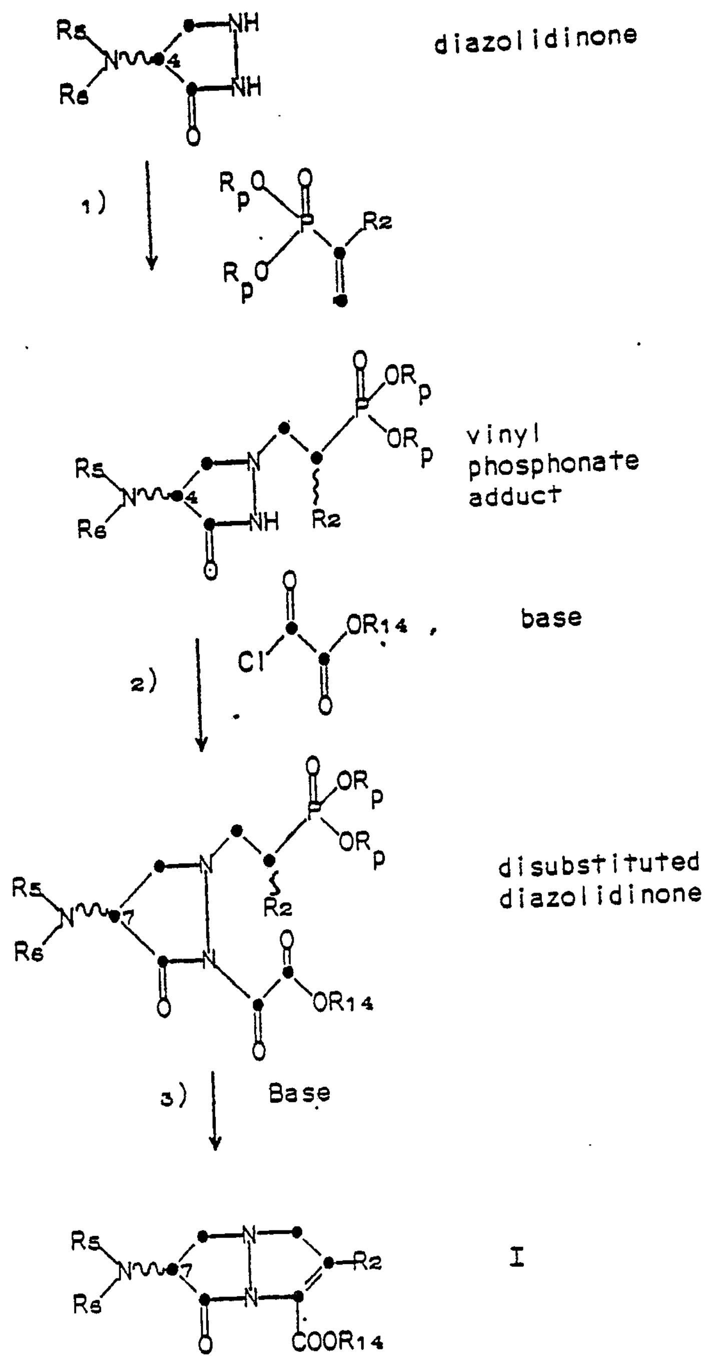


In the above Scheme, $R_p$ is $C_1$ to $C_6$ alkyl or phenyl. The variable $R_2$ is the same as for Formula I. The variable $R_{14}$ in the above Scheme is a carboxy-protecting group or a non-toxic, metabolically-labile ester-forming group. The variables $R_5$ and $R_6$ in the above Scheme are:

a) taken together to form a phthalimido group; or

b) different and are either hydrogen or an amino-protecting group.

In the above Scheme, it is preferred that any carboxy, amino and hydroxy be in the protected form.

The stereospecificity at $C_7$ of the bicyclic pyrazolidinone product (Formula I) of the above sequence of reactions is determined by the $C_4$ stereochemistry of the diazolidinone starting material. Thus, a 4-(S)-diazolidinone starting material will yield a 7-(S)-bicyclic pyrazolidinone product (Formula I).

The first reaction in the above Scheme 3 (Reaction 1) is the alkylation of the $C_1$-nitrogen of the diazolodinone ring with a vinyl phosphonate reagent. The usual stoichiometry of the alkylation is a 1:1 molar ratio of the two reactants, but an excess of either reactant can be used. The solvent for the alkylation is an alcoholic solvent such as methanol, ethanol or isopropanol. Methanol is the preferred solvent.

The alkylation is usually carried out from between about 0° C to about room temperature. The reaction is a very rapid one, requiring as little as 1 hour but occassionally up to 48 hours for completion.

The vinyl phosphonate adduct obtained from the alkylation reaction (Reaction 1) is then acylated at the 2-position nitrogen with an oxalate ester acid chloride (after deprotonation of the diazolidinone with di-(isopropyl)ethylamine). The acylation reaction yields the corresponding 1,2-disubstituted diazolidinone.

Approximately one molar equivalent of the amine base (or other suitable base as appreciated by one skilled in the art) and one molar equivalent or less of the oxalate reactant per equivalent of the adduct reactant are combined in the acylation reaction. The three reactants can be combined in any order. The usual order is to combine the adduct and the oxalate reactants then add the amine base.

The vinyl phosphonate adduct may be acylated in either chlorinated hydrocarbon or ether solvents. Methylene chloride is the preferred solvent.

The acylation reaction is often complete in as little as one hour. However, the reaction may need to be stirred at the appropriate temperature for as long as approximately 12 hours to reach completion.

At the time when the three reactants are being combined, the temperature of the acylation mixture should be maintained from approximately -78° C to approximately -50° C. The reaction mixture is often stirred for approximately 1 hour at this temperature then allowed to warm to room temperature with stirring.

The 1,2-disubstituted diazolidinone obtained from Reaction 2 is then cyclized to give the bicyclic pyrazolidinone intermediate of Formula I, as depicted in Reaction 3.

In the cyclization reaction an equimolar or greater amount of the sodium hydride base per equivalent of the diazolidinone reactant is used. Ethers, and in particular tetrahydrofuran, are the preferred solvents. The reactants are usually combined and stirred at approximately 0° C for approximately 15 minutes to approximately 1 hour. The reaction mixture can be stirred at room temperature for as long as 24 hours.

A preferred reaction sequence for the above Scheme 1 is to acylate the vinyl phosphonate adduct and then cyclize the product without isolating the 1,2-disubstituted diazolidinone. In this preferred combination reaction, the vinyl phosphonate adduct and the acylating reagent are combined in any order and in the stoichiometry discussed for the acylation reaction. The solvent for the combination reaction is the same as for the acylation alone, including the preference for methylene chloride. The combination reaction requires more equivalents of di(isopropyl)ethylamine base per equivalent of adduct starting material (at least 2 versus at least 1) to effect both the acylation and the subsequent cyclization. The combination reaction mixture is stirred for from approximately 15 minutes to approximately 18 hours from about 0° C to about room temperature.

The progress of Reactions 1, 2, and 3 in Scheme 1 is monitored by conventional chromatographic techniques (such as thin layer chromatography or analytical-scale high pressure liquid chromatography) or spectroscopic techniques (such as infrared spec trometry or nuclear magnetic resonance spectroscopy). Spectroscopic and chromatographic techniques may also be combined to monitor the progress of the reactions. When the monitoring technique(s) demonstrates that the reaction(s) are substantially complete, the products from the above reactions are isolated by conventional methods.

Another synthetic route to the compounds of the Formula I is centered around a carbene-insertion ring closure and is diagramed below in Scheme 2.

## Scheme 2

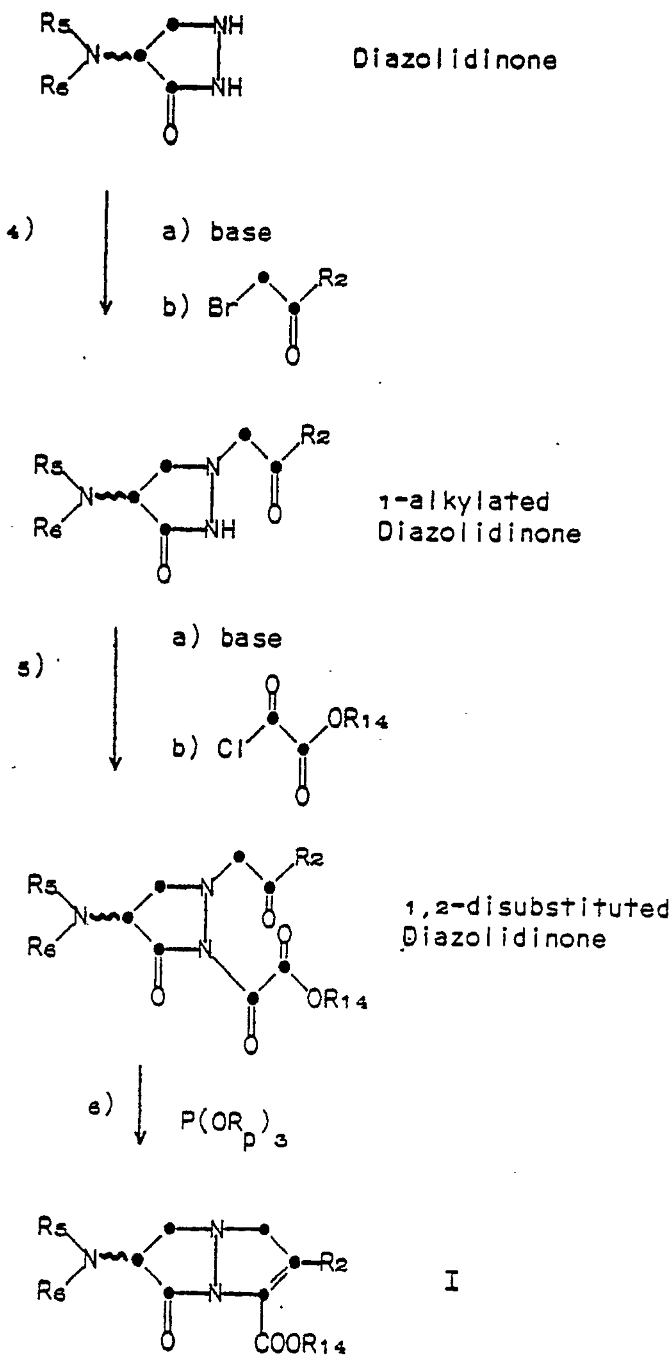


In the above Scheme 2, $R_2$ can be a group of the formula:

$$-\overset{(O)_z}{\overset{\|}{S}}-R_7$$

wherein Z is 0. $R_{14}$, $R_5$ and $R_6$ are the same as for the above Scheme 1. $R_p$ is $C_1$ to $C_6$ alkyl or phenyl, with ethyl being the preferred group.

The first reaction in Scheme 2 is the alkylation of $C_1$ nitrogen of the diazolidinone with an acetyl

fragment that bears the $R_2$ substituent. The first step of the alkylation is the deprotonation of the diazolidinone with a base chosen from sodium hydride, potassium t-butoxide, and the like. The diazolidinone and the base are preferably combined in a 1:1 molar ratio, but an excess of the diazolidinone is permissible.

The deprotonation step, as well as the subsequent alkylation step, is carried out in polar, aprotic solvents such as dimethylformamide, hexamethylphosphoramide, dimethylsulfoxide or dimethylacetamide. Dimethylformamide is the preferred solvent. When sodium hydride is the base, the reaction is stirred for between 1 to about 1.5 hours (to allow dissolution) then the alkylating agent is added. With the other bases, it is preferred to add the alkylating reagent within a few minutes after the addition of base. The deprotonation reaction mixture is stirred from between 0 °C to about room temperature, with 0 °C being the preferred temperature.

The deprotonated diazolidinone and the bromoacetyl alkylating reagent are combined in approximately a 1:1 molar ratio, although an excess of either reagent is permissable. The solvents for the alkylation step are the same as for the deprotonation step, and again dimethylformamide is the preferred solvent. The alkylation is generally complete after about 3 to about 24 hours and is stirred from about 0 °C to about room temperature.

The 1-alkylated diazolidinone obtained from Reaction 4 is acylated to yield the 1,2-disubstituted diazolidinone. The acylation reaction is depicted in the above Scheme as Reaction 5. The first step of Reaction 5 is a deprotonation reaction and the second step is the acylation of the resultant anion. The deprotonation step is preferably carried out with di(isopropyl)ethylamine present in an equimolar amount with the 1-alkylated diazolidinone reactant, although either reactant may be present in excess; further one skilled in the art will appreciate that other bases will be efficacious. The deprotonation reactants are combined in any of the chlorinated hydrocarbon solvents, although dichloromethane is preferred. The mixture is stirred from between about 0 °C to about 25 °C, with a range of between about 0 °C to about 10 °C being preferred.

Within a few minutes after the addition of the base, the oxalate ester acid chloride acylating agent is added to the mixture, again usually in an equimolar amount. A slight excess of the oxalate reactant may also be used. The oxalate reactant is generally added in a dropwise fashion over a period of approximately 20 minutes. The solvent for the acylation step is the same as the solvent of the deprotonation step. The temperature for the acylation step is the same as that for the deprotonation step, with approximately 10 °C being preferred. The acylation reaction will be complete after approximately 6 to approximately 48 hours, with the usual time being approximately 24 hours.

In the final reaction in Scheme 2 (Reaction 6) the 1,2-disubstituted diazolidinone is cyclized to a bicyclic pyrazolidinone of Formula I. A 5 to 10 molar equivalent excess of the phosphite reagent is combined with the diazolidinone reactant in either chloroform, 1,2-dichloroethyane, or an aromatic hydrocarbon solvent. Chloroform is the preferred solvent. The cyclization reaction mixture is heated to a range of about 50 °C to about 120 °C for between about 12 to about 72 hours. Twenty-four hours is a typical reaction time.

The progress of Reactions 4, 5, and 6 in Scheme 2 is monitored by conventional chromatographic techniques (such as thin layer chromatography or analytical-scale high pressure liquid chromatography) or spectroscopic techniques (such as infrared spectrometry or nuclear magnetic resonance spectroscopy). Spectroscopic and chromatographic techniques may also be combined to monitor the progress of the reactions. When the monitoring technique(s) demonstrates that the reactions are substantially complete, the products from the above reactions are isolated by conventional methods.

The stereochemistry at $C_7$ of the bicyclic pyrazolidinone product (Formula I) of the reaction sequence in Scheme 2 is determined by the stereochemistry at $C_4$ of the diazolidinone starting material. Thus, a 4-(S) diazolidinone will yield a 7-(S) bicyclic pyrazolidinone product.

As a further aspect of the present invention, there is provided a process for preparing compounds of Formula (I) wherein $R_2$ is keto, amino, and chloro, according to the following scheme:

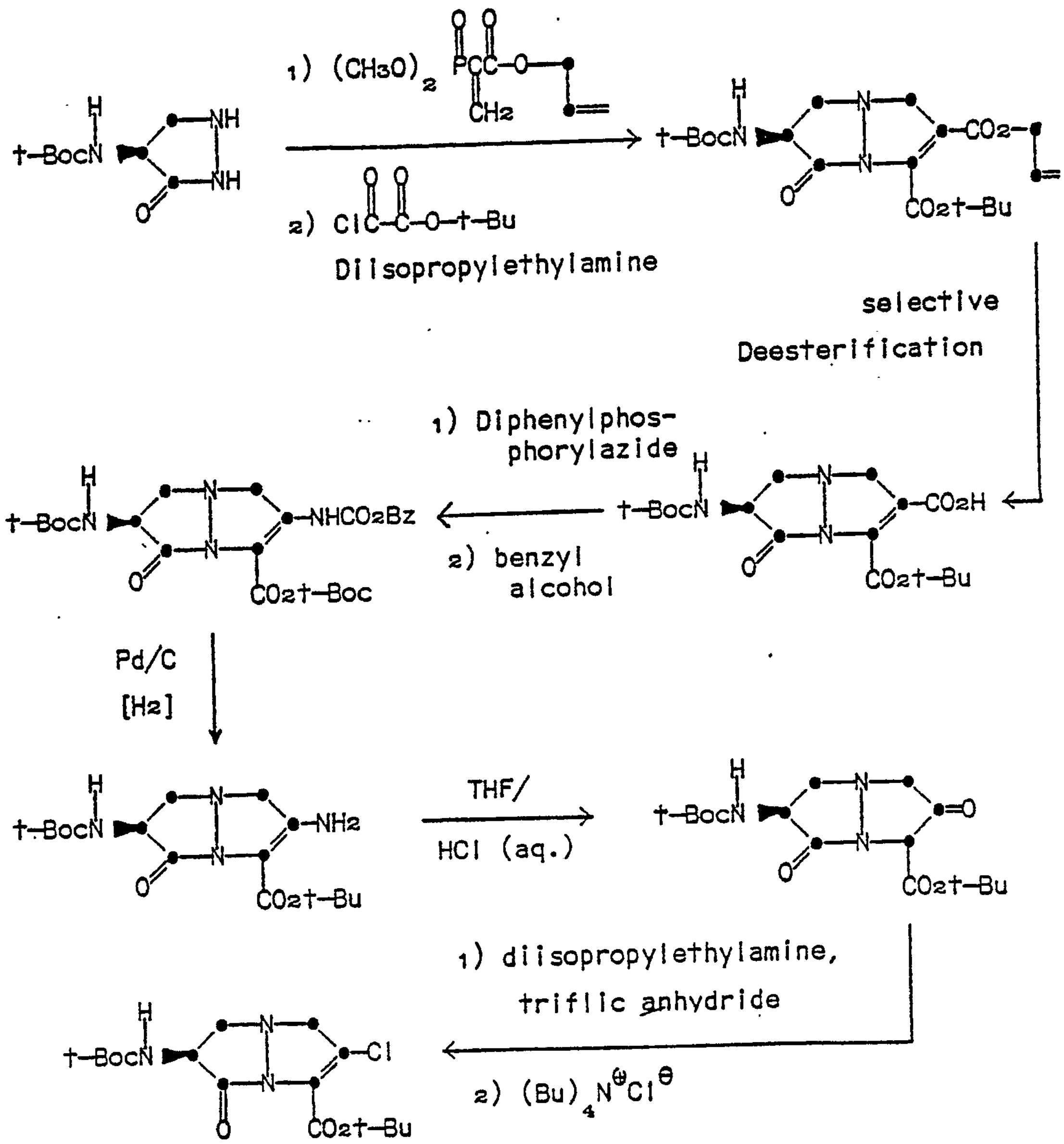


Accordingly, the 3-keto, 3-amino, and 3-carboxy compound can be used to further derivatize into other compounds. For example, the 3-keto compound can be reacted with triflic anhydride in the presence of a base such as diisopropylethylamine to form a 3-trifluoromethanesulfonyl derivative. This compound can then be reacted with a wide variety of nucleophiles to further derivatize the 3-position. Further, the 3-carboxy group or an active derivative thereof can be reacted with a wide variety of compounds, for example, thiols, and amines.

Further, the 3-isocyanate derivative formed in situ by reaction of the 3-carboxy derivative with diphenylphosphoryl azide and N-methylmorpholine can be reacted with sulfur, oxygen, and nitrogen nucleophiles to provide the compounds of the invention wherein ($R_1$ or) $R_2$ is $-NR_{12}R_{13}$ and one of $R_{12}$ or $R_{13}$ is hydrogen and the other is a group of the formula

$$-NH\overset{O}{\overset{\|}{C}}-Nu$$

wherein Nu is as defined above. For example, ethanethiol provides the compound of Formula (I) wherein $R_2$ (or $R_1$) is

$$-NH\overset{O}{\overset{\|}{C}}SCH_2CH_3.$$

Further reaction of the isocyanate with an alcohol provides the 3-carbamate.

In the above scheme, the 4-protected amino diazolidinone is reacted with a vinyl phosphonate and then N-acylated with t-butyl oxalyl chloride in the presence of base, followed by in situ ring closure to form the

3-carboxylic ester. Selection removal of the 3-ester by standard methodology provides the free carboxy group.

The 3-carboxy group can then be readily converted to the carbamate by acylation with diphenylphosphoryl azide in the presence of base and an alcohol followed by rearrangement to provide the carbamate.

The carbamate can then be reduced by catalytic hydrogenation to provide the free 3-amino compounds of the present invention. Of course, protection of the above groups may be desirable for subsequent functionalization of other portions of the bicyclic pyrazolidinone molecule.

The compounds of the present invention wherein $R_2$ is

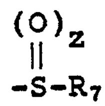

and $R_7$ is (disubstituted)amino can be also prepared by the following scheme):

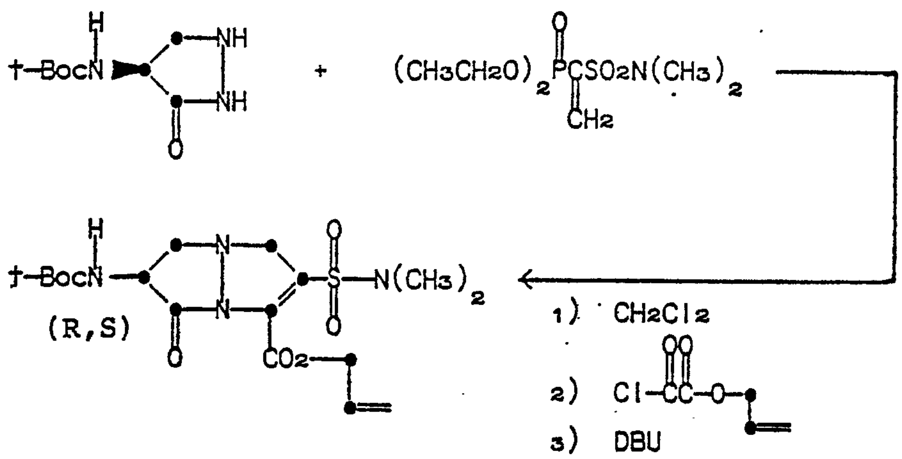


As will be appreciated by the scheme above, other esters of oxalyl chloride can be utilized to provide other esters of the bicyclic pyrazolidinone. Accordingly, other (disubstituted)amino sulfones can be utilized to provide the desired group at the 3-position (i.e., $R_7$).

The first step for the acylation of a 7-(protected amino) bicyclic pyrazolidinone compound ("7-protected amino nucleus") is the removal of the amino-protecting group. For example, the trimethylsilyl protecting group is removed by simple hydrolysis, the t-butoxycarbonyl group is removed by either acidic hydrolysis (with trifluoroacetic acid) or acidolysis (hydrochloric acid in glacial acetic acid), and the allyloxycarbonyl group is removed as a palladium complex. The conditions for the removal of other groups are well known in the cephalosporin and penicillin arts.

Removal of the acid-labile amino-protecting groups usually yields the 7-amino nucleus as a salt. The salt of the nucleus is neutralized by conventional procedures before acylation. For instance, the removal of the t-butoxycarbonyl group with trifluoroacetic acid leaves the trifluoroacetate salt of the resultant 7-amino nucleus. The salt is taken up in tetrahydrofuran and bis(trimethylsilyl)trifluoroacetamide is added to yield the corresponding (neutralized) 7-amino compound. The neutralized compound can either be isolated then acylated or acylated in situ.

The methods for the acylation of the neutralized 7-amino bicyclic pyrazolidinone with the acyl side chain are similar to the methods for the acylation of 6-aminopenicillanic acid, 7-aminodesacetoxycephalosporanic acid and 7-aminocephalosporanic acid. One method is to simply combine the 7-amino nucleus with an acid chloride or acid bromide in the presence of an acid scavenger. The acid chloride or acid bromide may be formed in situ. Another method is to combine the 7-amino nucleus with the free carboxylic acid form of the side chain (or its acid salt) and a condensing agent. Suitable condensing agents include N,N′-disubstituted carbodiimides such as N,N′-dicyclohexylcarbodiimide, N,N′-diethylcarbodiimide, N,N′-di-(n-propyl)carbodiimide, N,N′-di-(iso-propyl)carbodiimide, N,N′-diallylcarbodiimide, N,N′-bis(p-dimethylaminophenyl)carbodiimide, N-ethyl-N′-(4″-ethylmorpholinyl)carbodiimide, and the like. Other suitable carbodiimides are disclosed by Sheehan in U.S. Patent No. 2,938,892 and by Hofmann et al. in U.S.

Patent No. 3,065,224. Azolides, such as N,N′-carbonyldiimidazole and N,N′-thionyldiimidazol, may also be used as condensing agents. Dehydrating agents such as phosphorus oxychloride, the alkoxyacetylenes and 2-halogenopyridinium salts (such as 2-chloropyridinium methyl iodide, 2-fluoropyridinium methyl iodide, and the like) may be used to couple the free acid or its acid salt with the 7-amino nucleus.

Another acylation method entails first converting the free carboxylic acid form (or the corresponding salt) of the acyl side chain to the active ester derivative which is in turn used to acylate the nucleus. The active ester derivative is formed by esterifying the free acid form with groups such as p-nitrophenol, 2,4-dinitrophenol, trichlorophenol, pentachlorophenol, 2-chloro-4,6-dimethoxytriazene, N-chlorosuccinimide, N-chloro maleic imide, N-chlorophthalimide, 1-hydroxy-1H-benzotriazole or 1-hydroxy-6-chloro-1H-benzotriazole. The active ester derivatives can also be mixed anhydrides, which are formed with groups such as methoxycarbonyl, ethoxycarbonyl, isobutoxycarbonyl, trichloromethylcarbonyl and iso-but-2-ylcarbonyl and the carboxylic acid of the acyl side chain. The mixed anhydrides are synthesized by acylating the carboxylic acid of the acyl side chain.

Alternatively, the 7-amino nucleus can be acylated with the N-ethoxycarbonyl-2-ethoxy-1,2-dihydroquinoline (EEDQ) derivative of the acyl side chain. In general, the free acid form of the acyl side chain and EEDQ are reacted in an inert, polar organic solvent (such as tetrahydrofuran, acetonitrile, and the like). The resultant EEDQ derivative is used in situ to acylate the 7-amino nucleus.

The antimicrobial activity of the bicyclic pyrazolidinones acylated with the appropriate 7-acyl group is further enhanced on removal of any remaining amino, hydroxy, and/or carboxy protecting groups on the molecule. As discussed above, such removal methods are generally well known in the cephalosporin, penicillin, and peptide arts. Once the carboxy groups are deprotected, the non-toxic, metabolically-labile, ester-forming ("oral ester") group(s) may be put in place on the desired carboxy groups at $R_1$, $R_2$, $R_3$, and $R_4$. The methods for making the oral ester derivatives are well known in the cephalosporin and penicillin arts.

A $C_4$-racemic mixture of pyrazolidinium ylide starting materials for the reactions in Schemes 1 and 2 and a $C_4$-racemic mixture of starting materials for the reactions in Schemes 1 and 2 are synthesized according to the process depicted below in Scheme 3.

## Scheme 3

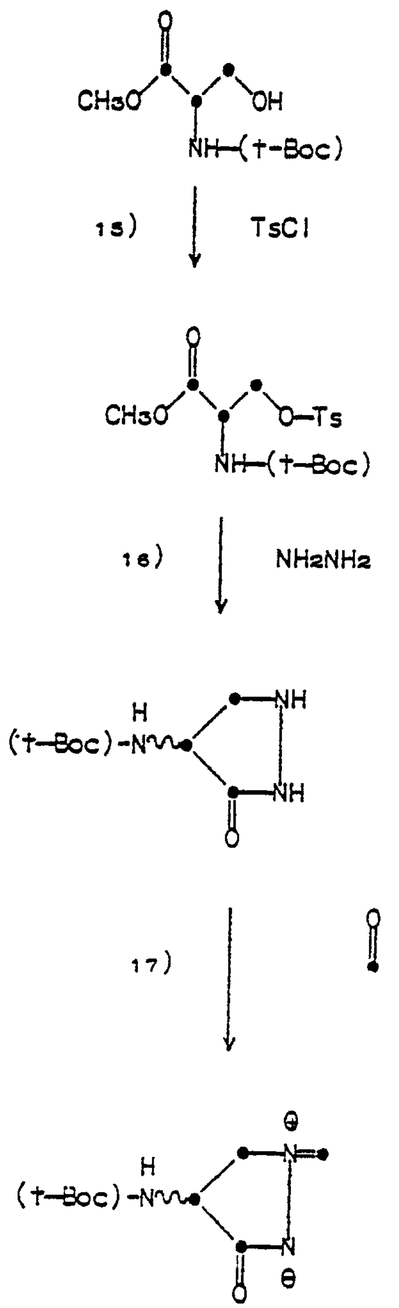


protected serine

tosyl serine

diazolidinone

ylide

The above Scheme depicts the synthesis of 4-(t-butoxycarbonylamino) starting materials. Starting materials with different amino-protecting groups are obtained by starting with a different protecting group on the protected serine derivative.

The first step in the synthesis of the starting materials, represented by Reaction 15 in the above Scheme, is the tosylation of the hydroxy group of the protected serine derivative. The tosylation is carried out in methylene chloride with p-toluenesulfonyl chloride in the presence of a catalytic amount of 4-dimethylaminopyridine and greater than one equivalent of pyridine. The reaction mixture is stirred at room temperature overnight.

The tosylated serine obtained is cyclized to give the diazolidinone. The cyclization represented by Reaction 16 is carried out by adding the tosyl serine to a solution of 97% hydrazine in methylene chloride under nitrogen. The mixture is then stirred at room temperature for five hours.

The final reaction in the synthesis of a $C_4$-racemic mixture of the pyrazolidinium ylide starting materials

comprises the condensation of a ketone or aldehyde with a diazolidinone. As a useful alternative procedure, the ketal of the ketone may be condensed with the diazolidinone in the presence of an acid. For example, the diazolidinone reagent is combined with acetone dimethyl acetal in methanol and then the solution is treated with d-10 camphorsulfonic acid. The mixture is refluxed for 1.5 hours to give the dimethyl ylide (i.e., $R_3$ and $R_4$ are methyl). The unsubstituted ylide (when $R_3$ and $R_4$ are hydrogen) is synthesized by combining the diazolidinone reagent and 37% aqueous formaldehyde in methanol and stirring the mixture for twenty minutes at room temperature. When $R_3$ and $R_4$ are different those skilled in the art will recognize that Reaction 17 will produce a mixture of E and Z isomers.

The stereospecific synthesis of $C_4$-chiral pyrazolidinium ylide and diazolidinone starting materials is diagramed below in Scheme 4.

## Scheme 4

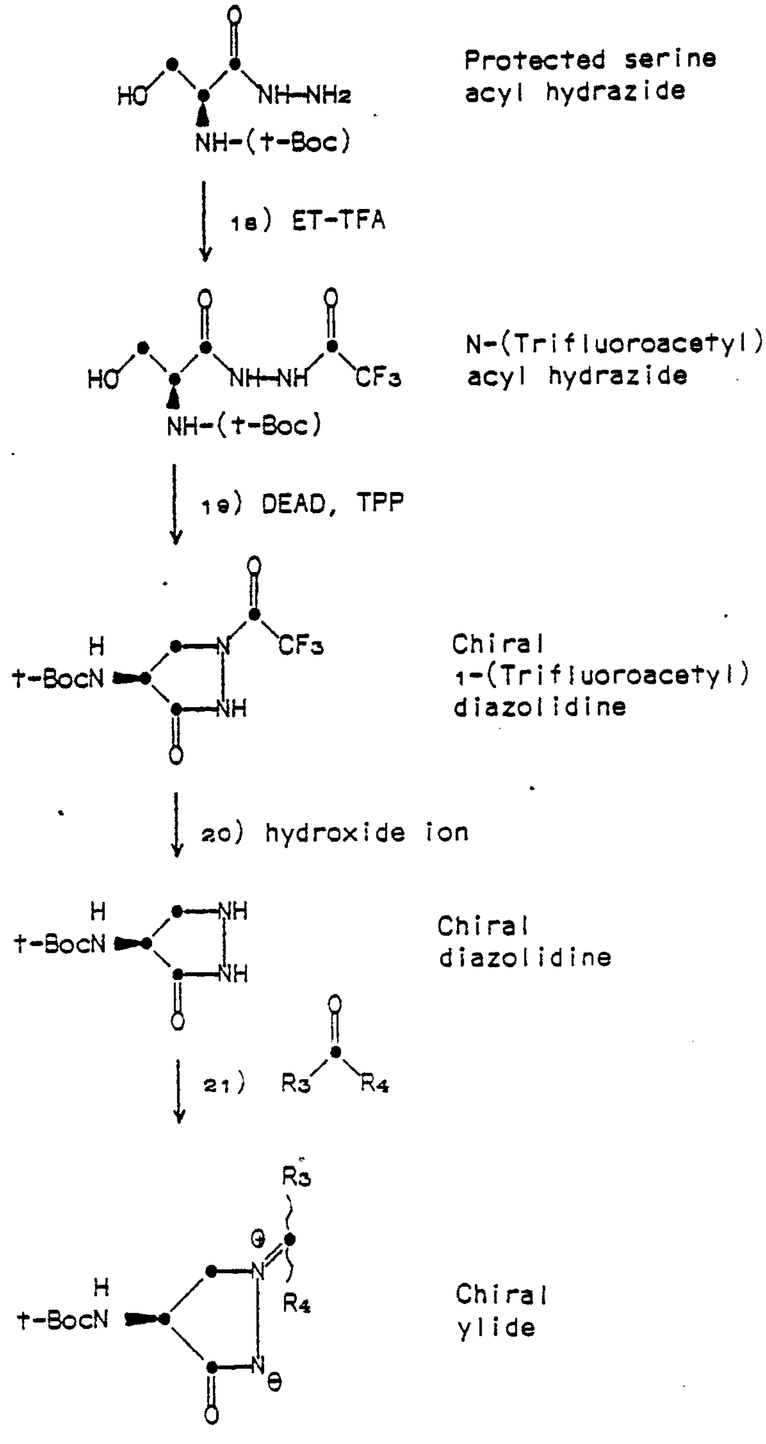


Protected serine acyl hydrazide

N-(Trifluoroacetyl) acyl hydrazide

Chiral 1-(Trifluoroacetyl) diazolidine

Chiral diazolidine

Chiral ylide

The above Scheme depicts the synthesis of chiral 4-(S)-(t-butoxycarbonylamino) ylide compounds. Ylide compounds with the 4-(R) configuration are synthesized by starting with the protected D-serine acyl hydrazide instead of the L-isomer depicted above. Both 4-(R) or 4-(S) compounds with amino-protecting groups other than t-butoxycarbonyl are synthesized from the corresponding serine enantiomer substituted with an amino-protecting group other than t-butoxycarbonyl.

The protected serine acyl hydrazide precursor of Scheme 4 is synthesized in a procedure analogous to B. Iselin and R. Schwyzer, Helv. Chim. Acta 44, p. 169 (1961). The precursor is then acylated with the trifluoroacetyl moiety, as set forth in Reaction 18 in the Scheme. The hydrazide precursor is acylated with an excess of ethylthio trifluorothioacetate ("ET-TFA") in ethanol. The reaction mixture is stirred at room

temperature for 65 hours.

The N-(trifluoroacetyl) acyl hydrazide obtained from Reaction 18 is cyclized with triphenylphosphine ("TPP") and diethyl azodicarboxylate ("DEAD"), as depicted above in Reaction 19.

The stoichiometry of the cyclization of Reaction 19 has the N-(trifluoroacetyl) acyl hydrazide, phosphine, and diethyl azodicarboxylate reagent present in at least approximately a 1:1:1 molar ratio. The reaction will proceed in the presence of molar excesses above this ratio of any of the reactants.

The cyclization is initiated by first combining (in any order) the solvent, the N-(trifluoro acetyl) acyl hydrazide, and the phosphine, and secondly adding the azodicarboxylate reagent.

The temperature of Reaction 19 is not a critical parameter. The cyclization can be carried out at a temperature from approximately the freezing point to approximately the reflux temperature of the solvent. The preferred temperature is approximately room temperature.

The duration of Reaction 19 can be from approximately five minutes to approximately twenty four hours. The progress of the cyclization can be monitored by standard methods (such as thin layer chromatography, high performance liquid chromatography, etc.) The process is stopped when the monitoring method demonstrates that the reaction is substantially complete.

The solvents for the cyclization are aromatic hydrocarbon solvents such as benzene, toluene or xylenes; ethers such as diethyl ether, tetrahydrofuran, or 1,4-dioxane; chlorinated hydrocarbons such as methylene chloride, chloroform, carbon tetrachloride, dichloroethane, or chlorobenzene; amides such as dimethylformamide and dimethylacetamide; and other solvents such as hexamethylphosphoramide. Tetrahydrofuran is the preferred solvent. It is also desirable, but not essential, to dry and deoxygenate the solvent before use in the process.

While Reaction 19 in the above Scheme depicts the use of diethyl azodicarboxylate, the dimethyl and di(iso-propyl)azodicarboxylate analogs can also be used in the reaction.

The chiral 1-(trifluoroacetyl)diazolidine obtained from Reaction 19 is deacylated with dilute sodium hydroxide solution. The deacylation is represented as Reaction 20 in the Scheme. The deacylation entails generally suspending the chiral 1-(trifluoroacetyl)diazolidine in water and adding at least two equivalents of a dilute aqueous solution of either sodium hydroxide or potassium hydroxide. For instance, a two-fold excess of 1M sodium hydroxide solution can be used. It is preferred to have the initial pH of the solution from between about 11 to about 12. The resultant solution can be stirred from about 10 minutes to about 3 hours at a temperature from about 10° C to about 25° C. When the reaction is substantially complete the reaction solution is neutralized by the addition of dilute acid, such as 1N hydrochloric acid.

The optimal reaction time for the deacylation can be determined by monitoring the progress of the reaction with conventional chromatographic methods (such as thin layer chromatography, high performance liquid chromatography, or column chromatography), or spectroscopic methods, (such as infrared spectroscopy, nuclear magnetic resonance spectrometry, and mass spectrometry) or a combination of both methods. A preferred reaction time is from between about 30 minutes to about 1.5 hours.

The final reaction of Scheme 4, wherein the chiral diazolidines are converted to the chiral pyrazolidinium ylides, is carried out using the con ditions described for the analogous reaction (Reaction 17) in Scheme 9.

The synthesis of the above diazolidine and pyrazolidinium ylide starting materials are further described by L.N. Jungheim and R. E. Holmes, European Patent Application No. 86303177.9 incorporated herein by reference.

The acetylene, ethylene, vinyl phosphonate, and bromoacetyl starting materials in Schemes 1, 2, 3 and 4, respectively, are made by methods known in the art and/or are commercially available. The synthesis of some of these starting materials are also described in the Experimental Section below.

The antimicrobial compounds of Formula 1 inhibit the growth of certain organisms pathogenic to man and animals. The antimicrobial compounds of Formula I are compounds wherein the various amino, hydroxy and/or carboxy-protecting groups have been removed and either $R_5$ or $R_6$ is an acyl group derived from a $C_1$ to $C_{30}$ carboxylic acid and the other is hydrogen. The antimicrobial activity can be demonstrated in vitro using standard tube-dilution techniques. These in vitro tests demonstrate that, in general, the 7-(S) isomers have better antimicrobial activity than either the corresponding 7-(R) isomers or a mixture of the two isomers. Representative pathogens which are sensitive to the antimicrobial compounds of Formula I include Staphylococcus aureus X1.1, Streptococcus pyogenes C203, Streptococcus pneumoniae Park, Hemophilus influenzae 76 (ampicillin resistant), Escherichia coli N10, Escherichia coli EC14, Escherichia coli TEM (β-lactamase producer), Klebsiella pneumoniae X26, Klebsiella pneumoniae KAE (β-lactamase producer), Klebsiella pneumoniae X68, Enterobacter aerogenes C32, Enterobacter aerogenes EB17, Enterobacter cloacae EB5 (non-β-lactamase producer), Salmonella typhi X514, Salmonella typhi B35, Serratia marcescens X99, Serratia marcescens SE3, Proteus morganii PR15, Proteus inconstans PR33, Providencia

rettgeri C24, Citrobacter freundii CF17, and the like.

The antimicrobial compounds of this invention are useful for the therapeutic or prophylactic treatment of infections in warm-blooded animals caused by gram-positive, gram-negative and acid-fast bacteria.

The antimicrobial compounds can be administered orally, parenterally (e.g. intravenously, intramuscularly or subcutaneously) or as a topical ointment or solution in treating bacterial infections of warm-blooded animals.

A further aspect of this invention is the pharmaceutical compositions of the antimicrobial compounds of Formula I. In particular, these pharmaceutical compositions are useful for the control of gram-positive and gram-negative bacterial infections and comprise a suitable vehicle and a therapeutically effective amount of the antimicrobial compounds of Formula I.

A preferred pharmaceutical composition is comprised of a therapeutically effective amount of the 7-(S) antimicrobial compounds of the formula and a suitable vehicle.

With regard to compositions for oral administration (such as tablets and capsules), the term "suitable vehicle" means common excipients such as binding agents, for example, syrup, acacia, gelatin, sorbitol, tragacanth, polyvinylpyrrolidine (Povidone), methylcellulose, ethylcellulose, sodium carboxymethylcellulose, hydroxypropylmethylcellulose, sucrose and starch; fillers and carriers, for example corn starch, gelatin, lactose, sucrose, microcrystalline cellulose, kaolin, mannitol, dicalcium phosphate, sodium chloride and alginic acid; disintegrators such as croscarmellose sodium, microcrystalline cellulose, corn starch, sodium starch glycolate, alginic acid and mutable wetting agents such as sodium lauryl sulfate; and lubricants such as magnesium stearate and other metallic stearates, stearic acid, silicone fluid, talc, waxes, oils and colloidal silica. Flavoring agents such as peppermint, oil of wintergreen, cherry flavoring or the like can also be used. It may be desirable to add a coloring agent to make the dosage form more aesthetically pleasing in appearance or to help identify the product. The tablets may also be coated by methods well known in the art.

The pharmaceutical compositions of the present invention may also be in the form of oral liquid preparations, which may be either a) aqueous or oily suspensions, solutions, emulsions or syrups; or b) a dry powder to be reconstituted with water or another suitable vehicle before use. When used in conjunction with such oral liquid preparations, the term "suitable vehicle" means conventional additives such as suspending agents, for example, sorbitol, syrup, methyl cellulose, glucose/sugar syrup, gelatin, hydroxyethylcellulose, carboxymethyl cellulose, aluminum stearate gel or hydrogenated edible oils, for example almond oil, fractionated coconut oil, oily esters, propylene glycol or ethyl alcohol; and preservatives such as methyl or propyl p-hydroxybenzoates or sorbic acid.

The pharmaceutical composition can also be for intravenous (IV) use. Specifically, a water soluble form of the antimicrobial compound can be dissolved in one of the commonly used intravenous fluids and administered by infusion. When used in conjunction with compositions for IV use, the term "suitable vehicle" means such fluids as physiological saline, Ringer's solution or 5% dextrose solution.

For intramuscular preparations a sterile formulation of a suitable salt form of the antimicrobial compound (for example, the hydrochloride salt or sodium salt) can be formulated with a "suitable vehicle". Examples of such sterile formulations are a suitable salt form either dissolved in a pharmaceutical diluent (for example, Water-for-Injection, physiological saline, 5% glucose) or suspended in an aqueous base or a pharmaceutically acceptable oil base (for example, an ester of a long chain fatty acid such as ethyl oleate).

Topical compositions can be formulated with "suitable vehicles" such as hydrophobic or hydrophilic bases. Such bases include ointments, creams or lotions.

Veterinary pharmaceutical compositions of the antimicrobial compounds may be administered in the feed or the drinking water of farm animals. Alternatively, the compounds can be formulated as intramammary prep arations with "suitable vehicles" such as long- or quick-release bases.

The antimicrobial compounds of Formula I can also be formulated in unit dosage form in sterile vials, sterile plastic pouches containing a port with a septum, or sterile, hermetically sealed ampoules. The antimicrobial compound (or the corresponding pharmaceutically-acceptable salt) may be a dry powder or in crystalline or lyophylized form. The amount of the antimicrobial compound per unit dosage may vary from about 250 milligrams to about 10 grams.

A "therapeutically effective amount" of the antimicrobial compounds of Formula I is from approximately 2.5 mg to about 50 mg of compound per kilogram of body weight. This amount generally totals from about 1 gram to about 12 grams per day for an adult human.

A further aspect of this invention is a method for treating or controlling infectious diseases caused by gram-positive and gram-negative organisms in warm-blooded animals. This method comprises administering to the infected host a therapeutically effective amount of the instant antimicrobial compounds. A typical daily dose for an adult human in this method is from about 0.5 grams to about 12 grams.

A preferred method comprises administering to an infected host a therapeutically effective amount of a 7-(S) antimicrobial compound of the formula

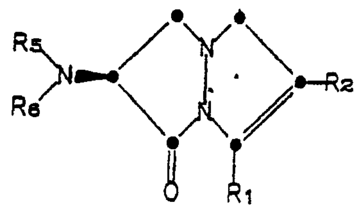

In practicing this method, the antimicrobial compound can be administered in a single daily dose or in multiple doses per day. The treatment regime may require administration over extended periods of time, for example, for several days or for from two to three weeks. The amount administered per dose or the total amount administered will depend on such factors as the nature and severity of the infection, the age and general health of the patient, and the tolerance to the antimicrobial compounds of Formula I of both the patient and the microorganism or microorganisms involved in the infection.

As a further aspect of this invention, there is provided a process for preparing a compound of Formula (I) which comprises

a) treatment of a compound of the formula

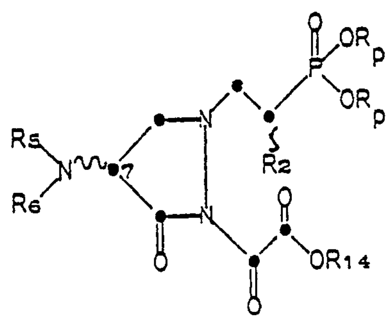

with a suitable base, wherein $R_p$ is $C_1$ to $C_6$ alkyl or phenyl; or

b) treatment of a compound of the formula

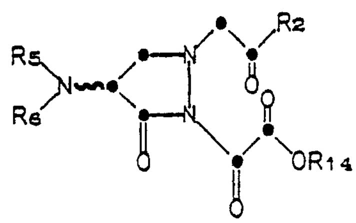

with a phosphite reagent of the formula P $(ORp)_3$, wherein Rp is $C_1$ to $C_6$ alkyl or phenyl; or

c) oxidizing a compound of Formula (I) wherein $R_2$ is

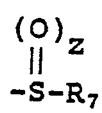

and Z is 0 or 1 to provide a compound of Formula (I) wherein Z is 2;

d) deprotection of carboxy and/or amino groups; or

e) acylation of a compound of the formula

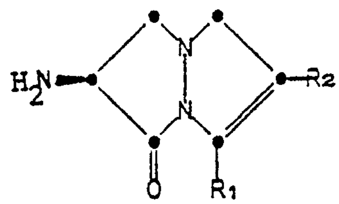

with an activated form of the carboxylic acid

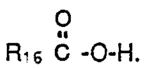

As yet a further aspect of the present invention, there is provided a process for preparing a compound of Formula (I) wherein $R_2$ is amino, which comprises hydrogenating a compound of the formula

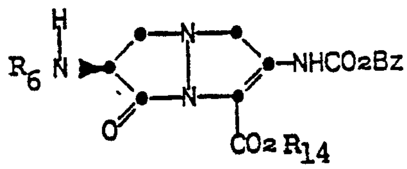

As yet a further aspect of the present invention, there is provided a process for preparing a compound of the formula

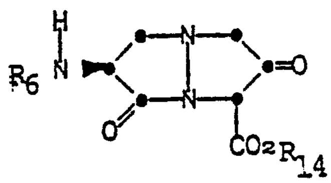

which comprises treating a compound of Formula (I), wherein $R_2$ is amino, with acid.

As yet a further aspect of the present invention there is provided a process for preparing a compound of Formula (I) wherein $R_2$ is

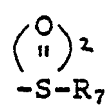

and $R_7$ is (disubstituted)amino, which comprises reacting compounds of the formula

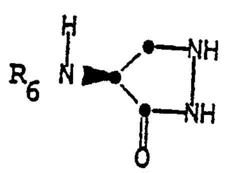 (a)

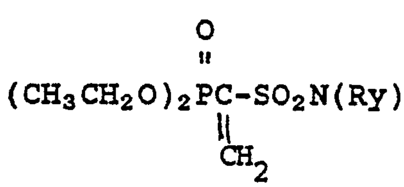 (b)

wherein Ry is phenyl, substituted phenyl, $C_1$ to $C_6$ alkyl, or $C_6$ to $C_{12}$ arylalkyl; and

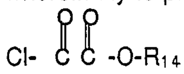

in an inert solvent in the presence of a strong base.

As yet a further aspect of the present invention there is provided a compound of the formula

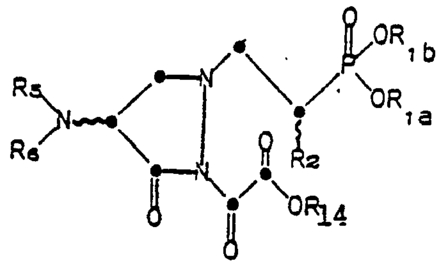

wherein $R_{1a}$ and $R_{1b}$ are the same or different and are $C_1$ to $C_6$ alkyl or phenyl; $R_{14}$ is a carboxy protecting group or a biological-labile ester; and $R_2$ is as defined in Claim 1.

As yet a further aspect of the invention there is provided a compound of the formula

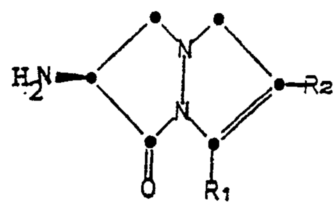

wherein $R_2$ is $C_6$ alkenyl or $C_2$ to $C_6$ alkyenyl or an acid-addition salt thereof.

The following Examples are provided to further illustrate the invention. It is not intended that the invention be limited in scope by reason of any of the following Preparations or Examples.

In the following Preparations and Examples, the terms melting point, nuclear magnetic resonance spectra, electron impact mass spectra, field desorption mass spectra, fast atom bombardment mass spectra, infrared spectra, ultraviolet spectra, elemental analysis, specific rotation, high performance liquid chromatography, and thin layer chromatography are abbreviated m.p., n.m.r., m.s., f.d.m.s., f.a.b.m.s., i.r., u.v., anal., o.r., HPLC, and TLC, respectively. In addition, the adsorption maxima listed for the i.r. spectra are only those of interest and not all of the maxima observed.

The abbreviations THF, DMF, TFA, and BSTFA stand for tetrahydrofuran, dimethylformamide, trifluoroacetate and N,O-bis(trimethylsilyl)trifluoroacetamide, respectively.

In conjunction with the n.m.r. spectra, the following abbreviations are used: "s" is singlet, "d" is doublet, "dd" is doublet of doublets, "t" is triplet, "q" is quartet, "m" is multiplet, "dm" is a doublet of multiplets and "br.s", "br.d", "br.t", and "br.m" are broad singlet, doublet, triplet, and multiplet respectively. "J" indicates the coupling constant in Hertz. "DMSO-$d_6$" is dimethyl sulfoxide where all protons have been replaced with deuterium.

The n.m.r. spectra were obtained on a Varian Associates EM-390 90 MHz or T-60 60 MHz instrument, on a Jeol FX-90Q 90 MHz instrument, on a Brüker Corp. 270 MHz instrument or on a General Electric QE-300 300 MHz instrument. The chemical shifts are expressed in $\delta$ values (parts per million downfield from tetramethylsilane). The field desorption mass spectra were taken on a Varian-MAT 731 Spectrometer using carbon dendrite emitters. Election Impact Mass Spectra were obtained on a CEC 21-110 instrument from Consolidated Electrodynamics Corporation. Infrared spectra were obtained on a Perkin-Elmer 281 instrument. Ultraviolet Spectra were obtained on a Cary 118 instrument. Specific rotations were obtained on a Perkin-Elmer Q-41 instrument. Thin layer chromatography was carried out on E. Merck silica gel plates. Melting points are uncorrected.

## Experimental Section

All of the starting materials used hereinafter are either commercially available, known per se in the art, or are described in Sigmund, et al., European Patent Application No. 86303174.6 (Publication No. 0202046).

Preparation 1

4-(R,S)-(t-Butoxycarbonylamino)-3-Oxo-1-[1′-Cyano-1′-(Diethylphosphonato)eth-2′-yl]-1,2-Diazolidine

Under a nitrogen atmosphere, 4-(R,S)-(t-butoxycarbonylamino)-3-oxo-1,2-diazolidine (22.7 g, 0.113 mol) was dissolved in methanol (200 ml) with heating and the solution was cooled to 0°C. 2-(Diethylphosphonato)acrylonitrile (0.113 mol) was washed into the solution with additional methanol (10 ml) and the solution was stirred at 0°C for 1 hour then at room temperature overnight and concentrated in vacuo to an oil. The oil was flash chromatographed on silica gel eluted with 5% methanol/ethyl acetate to yield 15.83 g of 4-(R,s)-(t-butoxycarbonylamino)-3-oxo-1-[1′-cyano1′-(diethylphosphonato)eth-2′-yl]-1,2-diazolidine: n.m.r. (90 MHz, $CDCl_3$): δ 1.16-1.40 (m, 6); 1.36 (s, 9); 3.08-4.80 (m, 10); 5.34 (br. s, 1); 8.72 (br. s, 1); i.r. ($CHCl_3$): 3021, 2250, 1712, 1264, 1023 $cm^{-1}$; f.a.b.m.s. (m/e): $M^+ + 1 = 391$.

Preparation 2

2-(Allyl Carboxylate)-3-Cyano-7-(R,S)-(t-Butoxycarbonylamino)-8-Oxo-1,5-Diazabicyclo[3.3.0]Octa-2-ene

Under a nitrogen atmosphere, 4-(R,S)-(t-butoxycarbonylamino)-3-oxo-1-[1′-cyano-1′-(diethyl phosphonato)eth-2′-yl]-1,2-diazolidine (15.83 g, 0.041 mol) was dissolved in methylene chloride (250 ml) and the solution was cooled to 0°C. Allyl oxalate acid chloride (6.03 g, 0.041 mol) then bis(isα-propyl)-ethylamine (10.49 g, 0.082 mol) were added, and the solution was stirred at 0°C for 1.5 hours, washed with water (2 x), dried over magnesium sulfate, filtered and concentrated to a yellow oil (16.46 g). The oil was flash chromatographed on silica gel eluted with 50% hexane in ethyl acetate to yield 3.97 g of 2-(allyl carboxylate)-3-cyano-7-(R,S)-(t-butoxycarbonylamino)-8-oxo-1,5-diazabicyclo[3.3.0]octa-2-ene. (The following data was gathered on a small amount of this final product that had been triturated with diethyl ether.) n.m.r. (300 MHz, $CDCl_3$): δ 1.47 (s, 9); 2.93 (dd, 1, J = 9 and 12); 3.93, 4.37 (ABq, 2, J = 12); 4.09 (br. t, 1, J = 9); 4.62-4.77 (m, 1); 4.78-4.96 (m, 2); 5.13 (br. s, 1); 5.36 (d, 1, J = 12); 5.47 (d, 1, J = 15); 5.92-6.06 (m, 1); i.r. ($CHCl_3$): 3040, 2990, 2220, 1753, 1742, 1716, 1501, 1407, 1370, 1160 $cm^{-1}$; f.d.m.s. (m/e): $M^+ = 348$; u.v. (EtOH): $\lambda_{max} = 212$ ($\epsilon = 8400$), 365 ($\epsilon = 5300$); Anal. Calcd for $C_{16}H_{20}N_4O_5$:
Theory: C, 55.17; H, 5.79; N, 16.08;
Found: C, 55.46; H, 5.56; N, 15.95.

Preparation 3

2-(Allyl Carboxylate)-3-Cyano-7-(R,S)-Amino-8-Oxo-1,5-Diazabicyclo[3.3.0]Octa-2-ene Hydrochloride

Under a nitrogen atmosphere, 2-(allyl carboxylate)-3-cyano-7-(R,S)-(t-butoxycarbonylamino)-8-oxo-1,5-diazabicyclo[3.3.0]octa-2-ene (1.54 g, 4.43 mmol) was combined with a glacial acetic acid solution that had a 3N concentration of anhydrous hydrogen chloride (45 ml) and the mixture was stirred at room temperature until it was a solution (5 minutes), when it was evaporated in vacuo to dryness. The residue was redissolved in methylene chloride and the solution was taken to dryness in vacuo two times to yield 2-(allyl carboxylate)-3-cyano-7-(R,S)-amino-8-oxo-1,5-diazabicyclo[3.3.0]octa-2-ene hydrochloride.

Preparation 4

2-(Allyl Carboxylate)-3-Cyano-7-(R,S)-[2-(2-Allyloxycarbonylaminothiazol-4-yl)-2-(Z)-Methoxyiminoacetamido]-8-Oxo-1,5-Diazabicyclo[3.3.0]Octa-2-ene

Under a nitrogen atmosphere, 2-[2′-(allyloxycarbonylamino)thiazol-4′-yl]-2-(Z)-methoxyiminoacetic acid (1.26 g, 4.43 mmol) was slurried in methylene chloride (25 ml) and the slurry was cooled to 0° C. 2-Chloro-4,6-dimethoxy-1,3,5-triazine (777 mg, 4.43 mmol) then N-methylmorpholine (0.487 ml, 4.43 mmol) was added and the mixture was stirred at 0° C for 70 minutes. 2-(allyl carboxylate)-3-cyano-7-(R,S)-amino-8-oxo-1,5-diazabicyclo[3.3.0]octa-2-ene hydrochloride (4.43 mmol) was dissolved in methylene chloride (20 ml) and was added followed by an additional amount of N-methylmorpholine (0.49 ml). The resultant mixture was stirred at 0° C for 1.5 hours then at room temperature overnight and concentrated to dryness in vacuo. The residue was flash chromatographed on silica gel eluted with ethyl acetate to yield 630 mg of a yellow oil. The oil was taken up in a mixture of ethyl acetate/hexane and the resultant precipitate was isolated by filtration to yield approximately 180 mg of 2-(allyl carboxylate)-3-cyano-7-(R,S)-[2-(2-allyloxycarbonylami nothiazol-4-yl)-2-(Z)-methoxyiminoacetamido]-8-oxo-1,5-diazabicyclo[3.3.0]octa-2-ene: n.m.r. (300 MHZ, DMSO-$d_6$): $\delta$ 3.20 (dd, 1, J = 12 and 12); 3.82-3.94 (m, 1); 3.88 (s, 3); 4.13, 4.41 (ABq, 2, J = 15); 4.70 (d, 2, J = 6); 4.85 (d, 2, J = 6); 5.02-5.15 (m, 1); 5.22-5.52 (m, 4); 5.86-6.04 (m, 2); 7.44 (s, 1); 9.29 (d, 1, J = 6); 12.18 (s, 1); i.r. ($CHCl_3$); 3020, 3000, 2230, 1733, 1681, 1554, 1411, 1371, 1276, 1044 $cm^{-1}$ ; f.d.m.s. (m/e): $M^+$ = 515; u.v. (EtOH) $\lambda_{max}$ = 209 ($\epsilon$ = 22387), 227 ($\epsilon$ = 21822), 263 ($\epsilon$ = 14291), 363 ($\epsilon$ = 5800);
Anal. Calcd for $C_{21}H_{21}N_7O_7S_1$ :
Theory: C, 48.93; H, 4.11; N, 19.02;
Found: C, 49.14; H, 4.23; N, 18.77.

## Preparation 5

2-(Carboxylic Acid)-3-Cyano-7-(R,S)-[2-(2-Aminothiazol-4-yl)-2-(Z)-Methoxyiminoacetamido]-8-Oxo-1,5-Diazabicyclo[3.3.0]Octa-2-ene

2-(Allyl carboxylate)-3-cyano-7-(R,S)-[2-(2-(allyloxycarbonylamino)thiazol-4-yl)-2-(Z)-methoxyiminoacetamido]-8-oxo-1,5-diazabicyclo[3.3.0]octa-2-ene (205 mg, 0.398 mmol) was dissolved in acetonitrile (10 ml) and the stirred solution was flushed with nitrogen. Triphenylphosphine (41.76 mg, 0.159 mmol) and palladium(II) acetate (4.47 mg, 0.0199 mmol) were added and the solution was stirred under nitrogen at room temperature for 20 minutes then cooled to 0° C. Tri(n-butyl)tin hydride (237.5 mg, 0.816 mmol, 0.22 ml) was added and the solution was stirred for ten minutes at 0° C and then at room temperature for one hour. Diethyl ether was added (10 ml) and the resultant solution was stirred for an additional hour at room temperature then additional ether (approximately 7 ml) and acetonitrile (approximately 1 ml) were added. 12 M hydrochloric acid (0.068 ml, 0.816 mmol) was added and the resultant yellow precipitate was collected by filtration, washed with diethyl ether (20 ml), dichloromethane (5 ml), and again with diethyl ether (10 ml) to yield 136 mg of a solid. The solid was chromatographed by medium pressure liquid chromatography on a $C_{18}$ reverse phase column eluted with 5% acetonitrile/1% acetic acid/water to yield approximately 15 mg of 2-(carboxylic acid)-3-cyano-7-(R,S)-[2-(2-aminothiazol-4-yl)-2-(Z)-methoxyiminoacetamido]-8-oxo-1,5-diazabicyclo[3.3.0]octa-2-ene: n.m.r. (300 MHz, DMSO-$d_6$): $\delta$ 3.00-4.00 (m, 1); 3.09 (dd, 1, J = 9 and 12), 3.84 (s, 3), 3.96 (d, J = 12, 1), 4.27 ($\frac{1}{2}$ ABq, 1, J = 12), 4.92-5.06 (m, 1), 6.98 (s, 1), 7.24 (br. s, 2), 9.15 (d, 1, J = 9); i.r. (KBr): 3320 (br), 2220, 1724, 1649, 1534, 1398, 1046 $cm^{-1}$; u.v. (EtOH): $\lambda_{max}$ = 227 ($\epsilon_{max}$ = 16225), 302 ($\epsilon_{max}$ = 9726); m.p. >225° C (d).

## Preparation 6

1-(Dimethylphosphonato)-1-(Methylsulfonato)Ethylene

Under a nitrogen atmosphere, benzene (50 ml), acetic acid (15 ml), paraformaldehyde (0.99 g, 0.033 mol), and pyrrolidine (0.234 g, 0.0033 mol) were combined and the mixture was warmed to reflux for 25 minutes then cooled to 0° C. 1-(Dimethylphosphonato)-1-(methylsulfonato)methane was added (5 g, 0.025 mol) and the solution was heated to reflux first for 10 minutes then a Dean-Stark trap was added to the apparatus and the solution was heated to reflux for 25 minutes. Toluene was added and the solution was concentrated in vacuo followed by the addition of toluene and reconcentration in vacuo to yield 1-(dimethylphosphonato)-1-(methylsulfonato)ethylene: n.m.r. (90 MHz, $CDCl_3$): δ 3.08 (s, 3); 3.80 (d, 6, J = 13); 6.80 (d, 1, J = 18); 7.00 (d, 1, J = 36).

## Preparation 7

### 4-(R,S)-(t-Butoxycarbonylamino)-3-Oxo-1-[1′-(Dimethylphosphonato)-1′-(Methylsulfonato)Eth-2′-yl]-1,2-Diazolidine

Under a nitrogen atmosphere, 4-(R,S)-(t-butoxycarbonylamino)-3-oxo-1,2-diazolidine (4.5 g, 22.4 mmol) was dissolved in methanol (40 ml) with warming then the solution was cooled to 0° C. 1-(Dimethylphosphonato)-1-(methylsulfonato)ethylene (25 mmol) was added and the solution was stirred for l hour at 0° C, for 1 hour and 50 minutes at room temperature then filtered. The solid collected by filtration was washed with methanol, dried in vacuo at room temperature overnight to give 3.25 g of 4-(R,S)-(t-butoxycarbonylamino)-3-oxo-1-[1′-(dimethylphosphonato)-1′-(methylsulfonato)eth-2′-yl]-1,2-diazolidine.
n.m.r. (300 MHz, DMSO-$d_6$): δ 1.40 (s, 9); 2.80-3.00 (m, 1); 3.10-3.66 (m, 3); 3.20 (s, 3); 3.66-3.80 (m, 6); 4.20-4.54 (m, 2); 7.20 (br. d, 1, J = 12); 9.70 (br. d, 1, J = 6); i.r. (KBr): 3310, 3190, 1710, 1696, 1525, 1232, 1165, 1142, 1052,1043 $cm^{-1}$; f.d.m.s. (m/e): $M^+$ = 415, 215 (M-200), 201 (M-215); m.p. 141-143° C;
Anal. Calcd for $C_{13}H_{26}N_3O_8S_1P_1$:
Theory: C, 37.59; H, 6.31, N, 10.12;
Found: C, 37.85; H, 6.30; N, 9.99.

## Preparation 8

### 2-(Allyl Carboxylate)-3-(Methylsulfonyl)-7-(R,S)-(t-Butoxycarbonylamino)-8-Oxo-1,5-Diazabicyclo[3.3.0]Octa-2-ene

Under a nitrogen atmosphere, 4-(R,S)-(t-butoxycarbonylamino)-3-oxo-1-[1′-(dimethylphosphonato)- 1′-(methylsulfonato)eth-2′-yl]-1,2-diazolidine (2.75 g, 6.6 mmol) was slurried in methylene chloride (150 ml) and the resulting slurry was cooled to 0° C. Allyl oxalate acid chloride (0.98 g, 6.6 mmol) was rinsed into the solution with additional methylene chloride then bis(iso-propyl)ethylamine (1.71 g, 13.2 mmol, 2.3 ml) was added and the mixture was stirred at 0° C for 30 minutes then at room temperature for 3.25 hours. The solution was washed with water, dried over magnesium sulfate, filtered and concentrated to yield 3.89 g of a yellow oil. The oil was combined with crude product from a previous procedure paralleling that above and the combination was flash chromatographed on silica gel eluted with 25% hexane/75% ethyl acetate to yield 1.87 g of 2-(allyl carboxylate)-3-(methylsulfonato)-7-(R,S)-(t-butoxycarbonylamino)-8-oxo-1,5-diazabicyclo[3.3.0]octa-2-ene. n.m.r. (90 MHz, $CDCl_3$): δ 1.44 (s, 9); 2.94 (dd, 1, J = 11 and 11); 3.10 (s, 3); 3.86-4.90 (m, 4); 3.97, 4.49 (ABq, 2, J = 12.5); 4.96-5.18 (br. d, 1, J = 5); 5.18-5.54 (m, 2); 5.68-6.18 (m, 1); i.r. ($CHCl_3$): 3021, 1741, 1717, 1326, 1142 $cm^{-1}$; f.d.m.s. (m/e): $M^+$ = 401; u.v. (EtOH): $\lambda_{max}$ = 329 (ε = 6037); m.p. 80° C.
Anal. Calcd for $C_{16}H_{23}N_3O_7S_1$ :
Theory: C, 47.87; H, 5.78, N, 10.47;
Found: C, 47.75; H, 5.74; N, 10.55.

## Preparation 9

4-(R,S)-(t-Butoxycarbonylamino)-3-Oxo-1-(1′-(Thien-2″-yl)-1′-Oxoeth-2′-yl)-1,2-Diazolidine

A DMF solution (150 ml) of 4-(R,S)-(t-butoxycarbonylamino)-3-oxo-1,2-diazolidine (12.06 g, 60 mmol) and sodium hydride (2.4 g, 60 mmol, 60% dispersion in mineral oil) were combined and the resultant mixture was stirred at room temperature for one hour then cooled to 0° C. A DMF solution (50 ml) of 2-(bromoacetyl)thiophene (12.9 g, 63 mmol) was added over a period of 15 minutes and the resultant mixture was stirred at 0° C. for approximately two to three hours, then stirred at room temperature for 18 hours. The reaction mixture was diluted with xylene (400 ml) and the solvents were removed in vacuo. The residue was dissolved in chloroform (400 ml) and water (200 ml). The chloroform layer was separated and the aqueous layer was extracted with chloroform (2 X, 200 ml). The chloroform layers were combined and washed with brine (3 X, 200 ml), dried over sodium sulfate, filtered and concentrated. in vacuo to yield a red oil (26 g). The oil was chromatographed by preparatory-scale high performance liquid chromatography on a silica gel column eluted with a gradient of 1:1 toluene:ethyl acetate to 100% ethyl acetate to yield approximately 10 g of a yellow foam. The foam was recrystallized from diethyl ether to yield 5.53 g of a solid. The solid was slurried in diethyl ether, filtered, and the collected solid was washed with diethyl ether to yield 4.46 g of 4-(R,S)-(t-butoxycarbonylamino)-3-oxo-1-[1′-(thien-2″-yl)-1′-oxoeth- 2′-yl]-1,2-diazolidine: n.m.r. (270 MHz, DMSO-$d_6$) δ 9.66 (s, 1), 8.06 (m, 2), 7.27 (d, 1), 7.14 (d, 1), 4.6 (m, 1), 4.28 (dd, 2), 3.5 (t, 1), 3.11 (t, 1), 1.37 (9); i.r. (KBr): 1719, 1685, 1657 $cm^{-1}$; f.d.m.s. (m/e): $M^+$ = 325.

## Preparation 10

2-(Carboxylic Acid)-3-Cyano-7-(S)-[2-(2-Aminothiazol-4-yl)-2-(Z)-Methoxyiminoacetamido]-8-Oxo-1,5-Diazabicycli[3.3.0]Octa-2-ene

In a manner similar to the synthesis of the corresponding 7-(R,S) compound (Preparation 2, and the intermediates thereto, Preparations 4, 3 and 2 herein, and Preparations 32, 31, 21 and 39 of Sigmund et al , European Patent Application No. 86303174.6 (Publication No. 0202046), the 4-(S) (chiral) substituted diazolidinone was used as the starting material, and the (chiral) title product was synthesized: n.m.r. (300 MHz, DMSO-$d_6$) δ 3.13 (dd, 1, J = 9 and 12), 3.85 (s, 3), 3.60-4.00 (m, 1); 4.03 and 4.34 (ABq, 2, J = 12), 4.94-5.10 (m, 1); 6.96 (s, 1), 7.25 (br. s, 2), 9.17 (d, 1, J = 9); i.r. (KBr): 3320 (br), 2220, 1724, 1642, 1534, 1399, 1047 $cm^{-1}$; u.v. (ethanol) $\lambda_{max}$=230 ($\epsilon_{max}$=16,345), 303 ($\epsilon_{max}$=9307; $[\alpha]_D^{25}$ = -383.1 (in methanol)-;.m.p. >225° (decompose).

## Preparation 11

2-(Allyl Carboxylate)-3-(Methylsulfonyl)-7-(R,S)-Amino-8-Oxo-1,5-Diazabicyclo[3.3.0]Octa-2-ene Hydrochloride

Under a nitrogen atmosphere, 2-(allyl carboxylate)-3-(methylsulfonyl)-7-(R,S)-(t-butoxycarbonylamino)-8-oxo-1,5-diazabicyclo[3.3.0]octa-2-ene (1.54 g, 4.43 mmol) was combined with a cold glacial acetic acid solution that had a 3N concentration of anhydrous hydrogen chloride (50 ml) and the mixture was stirred at room temperature until it was a solution (2 minutes), when it was evaporated in vacuo to dryness. The volatiles on the residue were removed by azeotropic distillation with methylene chloride (7X) then dried in vacuo at room temperature for 2.5 hours to yield 3.5 g of The residue was redissolved in methylene chloride and the solution was taken to dryness in vacuo two times to yield 2-(allyl carboxylate)-3-(methylsulfonyl)-7-(R,S)-amino-8-oxo-1,5-diazabicyclo[3.3.0]octa-2-ene hydrochloride.

## Preparation 12

2-(Allyl Carboxylate)-3-(Methylsulfonyl)-7-(R,S)-[2-(2-Allyloxycarbonylaminothiazol-4-yl)-2-(Z)-Methoxyiminoacetamido]-8-Oxo-1,5-Diazabicyclo[3.3.0]Octa-2-ene

Under a nitrogen atmosphere, 2-[2′-(allyloxycarbonylamino)thiazol-4′-yl]-2-(Z)-methoxyiminoacetic acid 2.7 g, 9.5 mmol) was suspended in methylene chlo ride (100 ml) and the suspension was cooled to 0°C. 2-Chloro-4,6-dimethoxy-1,3,5-triazine (1.7 g, 9.5 mmol) then N-methylmorpholine (1.0 ml, 9.5 mmol) was added and the mixture was stirred at 0°C for 45 minutes. 2-(allyl carboxylate)-3-(methylsulfonyl)-7-(R,S)-amino-8-oxo-1,5-diazabicyclo[3.3.0]octa-2-ene hydrochloride (3.2 g, 9.5 mmol) was added followed by an additional amount of N-methylmorpholine (1.0 ml, 9.5 mmol). The resultant mixture was stirred overnight first at 0°C then gradually warmed to room temperature and concentrated to dryness in vacuo. The residue was dissolved in a solution of ethyl acetate containing a 0.1N concentration of hydrochloric acid. The layers were separated and the organic layer was washed with saturated aqueous sodium bicarbonate solution and saturated aqueous sodium chloride solution, dried over magnesium sulfate, filtered and evaporated to dryness. The residue was flash chromatographed on silica gel eluted with ethyl acetate to yield 1.27 g, 23.5% of 2-(allyl carboxylate)-3-(methylsulfonyl)-7-(R,S)-[2-(2-allyloxycarbonylaminothiazol-4-yl)-2-(Z)-methoxyiminoacetamido]-8-oxo-1,5-diazabicyclo[3.3.0]octa-2-ene: n.m.r. (300 MHz, DMSO-$d_6$): δ 3.26 (s, 3); 3.20-3.30 (m, 1); 3.80-3.96 (m, 1); 3.90 (s, 3); 4.23 and 4.42 (ABq, 2, J = 12); 4.60-4.92 (m, 4); 5.04-5.20 (m, 1); 5.20-5.54 (m, 4); 5.86-6.06 (m, 2); 7.43 (s, 1); 9.31 (d, 1, J=6) 12.20 (s, 1); i.r. ($CHCl_3$); 3240, 3019, 1748, 1732, 1704, 1554, 1423, 1321, 1144 $cm^{-1}$: f.d.m.s. (m/e): $M^+ + 1$ = 569; u.v. (EtOH) $\lambda_{max}$ = 206 (ε = 22059), 264 (ε = 13351);
Anal. Calcd for $C_{21}H_{24}N_6O_9S_2$:
Theory: C, 44.36; H, 4.25; N, 14.78;
Found: C, 44.56; H, 4.25; N, 14.50.

## Preparation 13

2-(Carboxylic Acid)-3-(Methylsulfonyl)-7-(R,S)-[2-(2-Aminothiazol-4-yl)-2-(Z)-Methoxyiminoacetamido]-8-Oxo-1,5-Diazabicyclo[3.3.0]Octa-2-ene

Under a nitrogen atmosphere, palladium (II) acetate (54 mg, 0.24 mmol) was dissolved in acetone (13 ml), then triphenylphosine (316 mg, 1.2 mmol) was added. The resultant suspension was stirred at room temperature for 30 minutes. 2-(Allyl carboxylate)-3-(methylsulfonyl)-7-(R,S)-[2-(2-(allyloxycarbonylamino)-thiazol-4-yl)-2-(Z)-methoxyimino-acetamido]-8-oxo-1,5-diazabicyclo[3.3.0]octa-2-ene (1.23 g, 2.17 mmol) was dissolved in acetone (85 ml) and added to the above suspension. The resultant solution was stirred under nitrogen at room temperature for 40 minutes then cooled to -10°C for 10 minutes. Tri(n-butyl)tin hydride (1.26 g, 4.34 mmol, 1.17 ml) was added and the suspension was stirred for 75 minutes at -10°C and then at room temperature for 1.75 hours. (Over the course of the 1.75 hours, additional diethyl ether (40 ml) was added to the suspension). The suspension was cooled to -10°C and 1N hydrochloric acid (4.34 ml, 2 mmol) was added. The suspension was stirred at -10°C for 10 mintues then at room temperature for 10 minutes.

The resultant yellow precipitate was collected by filtration through a Celite® pad. The collected solid was washed with water (400 ml), and the wash was added to the acetone filtrate. The resultant cloudy yellow solution was filtered through a Celite pad and the filtrate was extracted with hexanes (4x, 150 ml). The filtrate was again filtered through a pad of Celite® then reduced to three-forths volume in vacuo The filtrate was again filtered through a Celite® pad then washed with diethyl ether (2x, 200 ml). The filtrate was concentrated in vacuo then lyophilized to give 1.0 g of a solid. A portion of the solid (100 mg) was chromatographed by medium pressure liquid chromatography on a silica gel column eluted with 5% methanol/0.5% acetic acid/water to give 30 mg of the title product. The remaining portion of the solid was chromatographed by medium pressure liquid chromatography on a (45 mm diameter x 12 inch length) silica gel column eluted with 2‰ methanol/0.5‰ acetic acid/water to give 2-(carboxylic acid)-3-(methylsulfonyl)-7-(R,S)-[2-(2-aminothiazol-4-yl)-2-(Z)-methoxyiminoacetamido]-8-oxo-1,5-diazabicyclo[3.3.0]octa-2-ene: n.m.r. (300 MHz, DMSO-$d_6$): 3.00-3.26 (m, 1), 3.22 (s, 3), 3.70-4.04 (m, 1), 3.84 (s, 3), 4.12 and 4.34 (ABq, 2, J=12), 4.94-5.10 (m, 1), 6.94 (s, 1); 7.24 (br. s, 2), 9.18 (d, 1, J=9); i.r. (KBr): 3340 (br), 1721, 1644, 1534, 1403, 1304, 1134, 1047 $cm^{-1}$ ; u.v. (EtOH): $\lambda_{max}$=232 ($\epsilon_{max}$=14681), 303 ($\epsilon_{max}$=11495); f.a.b. m.s. (m/e): $M^+ + 1$ = 445; m.p. >200°C.

Anal. Calc'd for $C_{13}H_{16}N_6O_7S_2$:
Theory: C, 37.83; H, 3.63; N, 18.91
Found : C, 37.57; H, 3.73; N, 18.68

## Preparation 14

2-(Carboxylic Acid)-3-(Methylsulfonyl)-7-(S)-[2-(2-Aminothiazol-4-yl)-2-(Z)-Methoximinoacetamido]-8-Oxo-1,5-Diazabicyclo[3.3.0]Octa-2-ene

In a manner similar to that of the Preparations above (except that the 4-(S)-diazolidinone is used as the starting material), the title product was synthesized: n.m.r. (300 MHz, DMSO-$d_6$): $\delta$ 3.13 (dd, J = 9, 12 Hz, 1H); 3.20 (s, 3H); 3.74-3.90 (m, 1H); 3.85 (s, 3H); 4.00 and 4.37 (ABq, J = 12 Hz, 2H); 4.93-5.05 (m, 1H); 6.95 (s, 1H); 7.23 (br s, 2H); 9.15 (d, J = 9 Hz, 1H); i.r. (KBr): 3320, 1722, 1652, 1534, 1402, 1383, 1304, 1133, 1046 $cm^{-1}$; m.s.: FAB m/e = 445 (m + 1); u.v.: (ethanol) $\lambda_{max}$ = 302 nm ($\epsilon$ = 10,022; 229 nm ($\epsilon$=17,163); o.r.: (DMSO) -88.84° at 589 nm;
Analysis Calc'd for $C_{13}H_{16}N_6O_7S_2 \cdot \frac{1}{2} H_2O$:
Theory: C, 37.08; H, 3.78; N, 18.53;
Found: C, 37.00; H, 3.64; N, 18.31.

## Examples 1 to 7

In a manner similar to that of Preparations 11 to 13 and as taught in Sigmund, et al, European Patent Application No. 86303174.6 (Publication No. 0202046) (except that the 4-(S)-diazolidinone was used as the starting material), the following compounds were synthesized. The $R_2$ substituent,

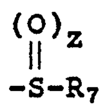

was varied by simply starting with the desired $R_7$ (and z) group as per Scheme 2, step 4 above.

The $R_{26}$ substituent was inserted by an alkylation of the free oxime (i.e., $R_{26}$ = hydrogen). Protection of the 2-amino group of the 2-aminothiazole-4-yl ring was protected throughout the synthesis with the triphenylmethyl group (trityl). The trityl group was then removed by the known triethylsilane/trifluoroacetic acid method. The 2-carboxyl group was protected throughout the synthesis as its allyl ester and eventually removed last by the palladium acetate/triphenylphosphine/triethylsilane method.

| Example | $R_{26}$ | $R_7$ | Physical Data |
|---|---|---|---|
| 1 | $-CH_2-CH=C(CH_3)_2$ | $-CH_3$ | 300 MHz ($DMSOd_6$): δ 1.65 (s, 3H); 1.72 (s, 3H); 2.95 (dd, 1H, J=10, 12 Hz); 3.18 (s, 3H); 3.72-3.80 (m, 1H); 3.81 and 4.13 (ABq, 2H, J=12 Hz); 4.55 (d, 2H, J=6 Hz); 4.89-5.01 (m, 1H); 5.35 (t, 1H, J=6 Hz); 7.06 (s, 1H); 7.22 (s, 2H); 9.04 (d, 1H, J=10 Hz); i.r. (KBr): 3541, 3335, 1715, 1649, 1536, 1412, 1383, 1321, 1302, 1230, 1135, 1001, 980 ($cm^{-1}$); ms: FAB m/e=499 ($M^+$ 1); u.v.: (EtOH) λ = 232 nm (ε=15,100); 302 nm (ε=11,700). |

| Example | $R_{26}$ | $R_7$ | Physical Data |
|---|---|---|---|
| 2 | $-CH_2CH_2CH_2CH=CH_2$ | $-CH_3$ | 300 MHz (DMSO$d_6$): δ 1.56-1.73 (m, 2H) 1.96-2.10 (m, 2H); 2.94 (dd, 1H, J=10 and 15 Hz); 3.00-3.46 (m, 1H); 3.14 (s, 3H); 3.72 (t, 1H, J=5 Hz); 4.00 (t, 2H, J=5 Hz); 4.12 and 3.88 (ABq, 2 H, J=10 Hz); 4.86-5.06 (m, 2H); 5.70-5.90 (m, 1H); 7.00 (s, 1H); 7.19 (br s, 2H); 9.02 (d, 1H, J=10 Hz); i.r. (KBr): 3323, 1718, 1651, 1533, 1401, 1321, 1303, 1135 $cm^{-1}$; m.s. (FD): m/e=455 ($M^+$ 1-$CO_2$(44)); u.v.: (EtOH)=303 nm, 235 nm; o.r.: (DMSO) -141.6° at 589 nm. |
| 3 | -allyl | $-CH_3$ | 300 MHz (DMSO$d_6$): δ 2.95 (dd, 1H, J=15, 10 Hz); 3.15 (s, 3H); 3.75 (t, 1H, J=10 Hz); 3.80 and 4.12 (ABq, 2H, J=10 Hz); 4.55 (d, 2H, J=5 Hz); 4.87-5.00 (m, 1H); 5.15 (d, 1H, J=10 Hz); 5.28 (d, 1H, J=12 Hz); 5.83-5.98 (m, 1H); 7.05 (s, 1H); 7.22 (br s, 2H); 9.08 (d, 1H, J=10 Hz); i.r. (KBr): 3410, 2920, 1730, 1717, 1533, 1413, 1303, 1135 $cm^{-1}$; m.s. (FAB): m/e=471 ($M^+$ 1); u.v.: (EtOH) λ=302 nm (ε=11797), λ=232 nm (ε=15342); o.r.: (DMSO) -62.8° at 589 nm. |

| Example | $R_{26}$ | $R_7$ | Physical Data |
|---|---|---|---|
| 4 | $-CH_2-C\equiv CH$ | $-CH_3$ | 300 MHz ($DMSOd_6$): δ 2.93 (dd, 1H, J=15, 10 Hz); 3.13 (s, 3H); 3.45 (t, 1H, J=2.5 Hz); 3.75 (t, 1H, J=10 Hz); 3.77 and 4.08 (ABq, 2 Hz, J=10 Hz); 4.65 (d, 2H, J=2.5 Hz); 4.85-4.97 (m, 1H); 7.12 (s, 1H); 7.25 (br s, 2H); 9.12 (d, 1H, J=10 Hz); i.r. (KBr): 3390, 2140, 1730, 1675, 1610, 1564, 1537, 1449, 1421, 1310 $cm^{-1}$; u.v.: (εtOH) λ=299 nm (ε=4170), λ=230 nm (ε=8020). |
| 5 | -allyl | $-CH_2CH_2CH_3$ | 300 MHz ($DMSOd_6$): δ 0.93 (t, 3H, J=10 Hz); 1.61 (q, 2H, J=10 Hz); 2.93 (dd, 1H, J=15, 10 Hz); 3.15-3.43 (m, 2H); 3.72 and 4.10 (ABq, 2H, J=10 Hz); 3.72 (t, 1H, J=6 Hz); 4.53 (d, 2H, J=5 Hz); 4.85-5.00 (m, 1H); 5.13 (d, 1H, J=10 Hz); 5.27 (d, 1H, J=20 Hz); 5.83-5.98 (m, 1H); 7.03 (s, 1H); 7.20 (br s, 2H); 9.07 (d, 1H, J=10 Hz); i.r. (KBr); 3420, 2980, 1717, 1658, 1534, 1411, 1380, 1280, 1129 $cm^{-1}$; m.s. (FAB): m/e=499 (M+); u.v.: (εtOH) λ=304 nm (ε=12100), λ=233 nm (ε=15300); o.r.: (DMSO) -17.8° at 589 nm. |

| Example | $R_{26}$ | $R_7$ | Physical Data |
|---|---|---|---|
| 6 | -allyl | -allyl | 300 MHz ($DMSOd_6$): δ 2.92 (dd, 1H, J=15, 10 Hz); 3.70 and 4.13 (ABq, 2H, J=10 Hz); 3.75 (t, 1H, J=10 Hz); 4.02-4.23 (m, 2H); 4.58 (d, 2H, J=7.5 Hz); 4.90-5.02 (m, 1H); 5.13-5.47 (m, 4H); 5.70-5.85 (m, 1H); 5.85-6.03 (m, 1H); 7.07 (s, 1H); 7.25 (br s, 2H); 9.13 (d, 1H, J=10 Hz); i.r. (KBr): 3450, 1735, 1653, 1534, 1440, 1410, 1321, 1130 $cm^{-1}$; m.s. (FAB): m/e=497 (M+); u.v.: (εtOH) λ=306 nm (ε=11600), λ=232 nm (ε=16100); o.r.: (DMSO) -149.6° at 589 nm. |
| 7 | $-CH_2CH_2CH=CH_2$ | $-CH_3$ | 300 MHz ($DMSOd_6$): δ 2.27-2.38 (m, 2H); 2.90-3.00 (m, 1H); 3.15 (s, 3H); 3.72 (t, 1H, J=10 Hz); 3.82 and 4.10 (ABq, 2H, J=10 Hz); 4.03 (t, 2H, J=7.5 Hz); 4.87-5.13 (m, 3H); 5.68-5.88 (m, 1H); 7.00 (s, 1H); 7.22 (br s, 2H); 9.02 (d, 1H, J=10 Hz); i.r. (KBr): 3360, 1717, 1645, 1533, 1413, 1406, 1303, 1135 $cm^{-1}$; m.s. (FAB): m/e=485 (M+); u.v.: (εtOH) λ=303 nm (ε=11800), λ=233 nm (ε=14900); o.r.: (DMSO) -163.4° at 589 nm. |

Examples 8 to 18

In a manner similar to that of Examples 100, 101, 102, 103, 121, and Preparation 39 (for the synthesis of the chiral (S) diazolidinone of Preparation 39) in Sigmund et al.,European Patent Application No. 86303174.6, Publication No. 0202046, the following compounds were synthesized. The same amino and carboxy protecting groups were utilized as described in Examples 1 8.

Example 8

7-(S)-[2-(2-Aminothiazol-4-yl)-2-(Z)-(allyl)oximinoacetamido]-3-cyano-8-oxo-1,5-diazabicyclo[3.3.0]octa-2-ene-2-carboxylic acid

NMR: 300 MHz (DMSO $d_6$): $\delta$ 2.99 (dd, 1H, J = 10, 12 Hz); 3.76 (t, 1H, J = 10 Hz); 4.09 and 3.72 (ABq, 2H, J = 10 Hz); 4.57 (d, 2H, J = 6 Hz); 4.86-5.06 (m, 1H); 5.17 (d, 1H, J = 9 Hz); 5.23 (d, 1H, J = 18 Hz); 5.96-6.02 (m, 1H); 7.06 (s, 1H); 7.23 (s, 2H); 9.12 (d, 1H, J = 8 Hz).
IR (KBr): 3410, 2220, 1720, 1652, 1609, 1534, 1407, 1322, 1247, 1205, 1120, 1019, 934 $cm^{-1}$.
Mass Spectra (FAB): m/e = 418 ($M^+$ + 1)
UV: (Ethanol) $\lambda$ = 232 nm (e = 14,400); 301 nm ($\epsilon$ = 8670)
OR: -240° at 589 nm (DMSO)

Example 9

7-(S)-[2-(2-Aminothiazol-4-yl)-2-(Z)-(2-carboxy-prop-2-yl)oximinoacetamido]-3-cyano-8-oxo-1,5-diazabicyclo-[3.3.0]octa-3-ene-2-carboxylic acid

NMR: 300 MHz (DMSO $d_6$): $\delta$ 1.34 (s, 3H); 1.38 (s, 3H); 3.13 (dd, 1H, J = 10, 12 Hz); 3.63-3.76 (m, 1H); 3.69 and 4.05 (ABq, 2H, J = 12 Hz); 4.86-5.00 (m, 1H); 6.80 (s, 1H); 7.15 (s, 1H).
UV: (Ethanol) $\lambda$ = 215 nm ($\epsilon$ = 16,600); 305 nm ($\epsilon$ = 8290)
OR: -175.299° at 589 nm (DMSO)

Preparation 15

t-Butyl-7-(S)-(t-butoxycarbonylamino)-3-carboxy-8-oxo-1,5-diazabicyclo[.3.3.0]octa-2-ene-2-carboxylate

A 7.64 g (38 mMol) sample of 4-(S)-(t-butoxycarbonylamino)-1,2-diazolidine-3-one was suspended in 350 ml of $CH_2Cl_2$, cooled in an ice bath under $N_2$, and treated with 14.03 g of a crude mixture containing approximately 9.26 g (42 mMol) of 1-(allyloxycarbonyl)-1-dimethylphosphonato-ethene.

The reaction mixture was stirred overnight and allowed to warm to room temperature. The reaction mixture was then cooled in an ice bath and treated with 7.60 g (46 mMol) of oxalyl chloride and 9.6 ml of diisopropylethylamine (dropwise addition) in 40 ml of $CH_2Cl_2$. After about 30 minutes, an additional 8.5 ml of diisopropylethylamine was dissolved in 40 ml of $CH_2Cl_2$ and added dropwise. After stirring for an additional 5 h at room temperature, the $CH_2Cl_2$ was removed in vacuo and the crude product redissolved in 300 ml of ethyl acetate.

The ethyl acetate solution was then washed sequentially with 150 ml saturated $NaHCO_3$, 1N HCl (2 x 150 ml), brine (3 x 150 ml) and dried over anhydrous $Na_2SO_4$. Removal of solvent in vacuo and

preparative-scale liquid chromatography (normal phase silica gel) provided 9.27 g (58% of t-butyl-7-(S)-(t-butoxycarbonylamino)-3-allyloxycarbonyl-8-oxo-1,5-diazabicyclo[3.3.0]octa-2-ene-3-carboxylate.

OR: -387.1° (methanol) 589 nm

Mass Spectra (FD): m/e = 423

NMR: 90 MHz ($CDCl_3$): δ 5.9, m, 1H; 5.2, bd, 2H, J = 7 Hz; 5.08, m, 3H; 4.8, m, 3H; 4.3, d, 1H, J = 13; 3.98, t, 1H, J = 8; 3.94, d, 1H, J = 13; 2.8, dd, 1H, J = 7, J = 10; 1.58, s, 9H; 1.44, s, 9H.

Under a nitrogen atmosphere, a 0.87 g (3.9 mM) sample of palladium acetate and 5.06 g (19.3 mM) sample of triphenylphosphine was combined in 250 ml of $CH_3CN$ and stirred for 20 min. A 20.51 g (48.5 mMol) sample of the 3-allyl carboxylate prepared above was dissolved in 350 ml of $CH_3CN$ and added to the reaction mixture and stirred for 35 min. The reaction mixture was then cooled to about 0° C and treated with 8.5 ml (53.3 mM) of triethylsilane. Stirring was continued for 40 min. and the reaction mixture was allowed to warm to room temperature over a $5\frac{1}{2}$ h period. The reaction mixture was then cooled in an ice bath and treated with 245 ml of 0.2N HCl and stirred for 5 min. The crude mixture was then diluted with 1.5 l of ethyl acetate and washed with 500 ml $H_2O$ (and brine). The aqueous washes were back-extracted with ethyl acetate and the combined organics washed with brine (2 x 500 ml), dried over anhydrous $MgSO_4$, and evaporated to provide 26 g of crude product. Preparative-scale liquid chromatography yielded 15.46 g of the title compound (83%).

OR: -319.0° at 589 nm (methanol)

NMR: 90 MHz ($CDCl_3$): δ 5.15, d, 1H, J = 6 Hz; 4.7, m, 1H; 4.36, d, 1H, J = 12 Hz; 4.01, dd, 1H, J = 7 Hz, J = 9 Hz; 3.84, d, 1H, J = 12 Hz; 2.84, dd, 1H, J = 9 H, J = 11 Hz; 1.56, s, 9H; 1.44, s, 9H.

## Example 10

t-Butyl-7-(S)-(t-butoxycarbonylamino)-3-amino-8-oxo-1,5-diazabicyclo[3.3.0]octa-2-ene-2-carboxylate

A. Under a nitrogen atmosphere, a 14.31 g (37.4 mMol) sample of the 3-carboxy nucleus synthesized in Example 171 was dissolved in 150 ml of $CH_2Cl_2$ and 1 l of benzene. The solution was then treated with 8.9 ml (1.1 molar equivalents) of diphenylphosphoryl azide and 7.2 ml (1.1 molar equivalents) of diisopropylethylamine and refluxed for 1 h. The reaction mixture was then treated with 5.4 ml (1.4 molar equivalents) of benzyl alcohol and refluxed for 3 h.

The volume was reduced in vacuo to remove the $CH_2Cl_2$ and washed sequentially with $H_2O$ (2 x 800 ml), saturated $NaHCO_3$ (500 ml), 0.2NHCl (2 x 500 ml) and brine (2 x 500 ml) and dried over anhydrous $MgSO_4$. The crude product solution was filtered and the solvent removed in vacuo to provide 20.4 g of crude product. Preparative scale liquid chromatography provided 7.59 g (42%) of t-butyl-7-(S)-(t-butoxycarbonylamino)-3-benzyloxycarbonylamino-8-oxo-1,5-diazabicyclo[3.3.0]octa-2-ene-3-carboxylate.

NMR: 90 MHz ($CDCl_3$): δ 9.05, bs, 1H; 7.28, s, 5H; 5.1, m, 1H; 5.08, s, 2H; 4.8, d, 1H, J = 14 Hz; 4.55, m, 1H; 4.02, dd, 1H, J = 9 Hz, J = 11 Hz; 3.96, d, 1H, J = 14 Hz; 2.66, dd, 1H, J = 8 Hz, J = 11 Hz; 1.5, s, 9H; 1.4, s, 9H.

B. A 3.94 g (8.07 mMol) sample of the compound prepared above in Part A was dissolved in 70 ml of ethyl acetate and hydrogenated for 90 min. at 48 psi of $H_2$ using 3.0 g of 5% Pd/C as catalyst. The catalyst was filtered off and the solvent removed in vacuo to provide 2.72 g (95% yield) of the title compound.

NMR: 90 MHz ($CDCl_3$: δ 5.48, bs, 2H, 5.24, m, 1H; 4.5, m, 1H; 4.04, t, J = 7 Hz; 3.92, d, 1H, J = 14 Hz; 3.56, d, 1H, J = 14 Hz; 2.6, dd, 1H, J = 9 Hz, J = 11 Hz; 1.5, s, 9H; 1.4, s, 9H.

OR: +10.7° at 589 nm (methanol)

## Example 11

t-Butyl-7-(S)-(t-butoxycarbonylamino)-3,8-dioxo-1,5-diazabicyclo[3.3.0]octane-2-carboxylate

A 790 mg (2.2 mMol) sample of the 3-amino nucleus prepared in Example 172 was dissolved in 27 ml of tetrahydrofuran, cooled in an ice bath and treated with 54 ml of 0.01N HCl. The pH of the solution was adjusted to pH 2.3 with 1N HCl. After about 12 min. the pH was adjusted to 5.3 with $NaHCO_3$, diluted with

brine and extracted with $CHCl_3$ (5 x 100 ml). The combined organics were washed with $NaHCO_3$ (150 ml), 0.2N HCl (2 x 150 ml), brine (3 x 150 ml), dried over $MgSO_4$ and filtered and the solvent was removed in vacuo to provide 539 mg of the title compound.
Elemental Analysis:
Theory: C, 54.07; H, 7.09; N, 11.82
Found : C, 54.31; H, 7.26; N, 11.78
Mass Spectra (FD): m/e = 355
NMR: 90 MHz ($CDCl_3$: $\delta$ 5.12, bd, 1H; 4.8, s, 1H; 4.64, m, 1H; 4.64, m, 1H; 4.32, T, 1H, J = 9 Hz; 3.82, d, 1H, J = 15 Hz; 3.16, d, 1H, J = 15 Hz; 2.86, T, 1H, J = 9 Hz; 1.5, s, 9H; 1.46, s, 9H.
OR: +76.4° at 589 nm (methanol)

## Example 12

7-(S)-[2-(2-Aminothiazol-4-yl)methoxyiminoacetamido]-3-methoxycarbonylamino-8-oxo-1,5-diazabicyclo-[3.3.0]octa-2-ene-2-carboxylic acid

The protected 3-methoxycarbonylamino nucleus prepared in Example 174 was treated with acetic acid/HCl (3.4 Mol) to provide the zwitterion.

Under a nitrogen atmosphere, a 0.64 mMol sample of 7-(S)-amino-3-methoxycarbonylamino-8-oxo-1,5-diazabicyclo[3.3.0]octa-2-ene-2-carboxylic acid was suspended in 10 ml of $CH_3CN$ and cooled in an ice bath. The nucleus suspension was then treated with 0.15 ml of diisopropylethylamine followed by 0.24 ml of MSTFA. The reaction solution was then treated with 224 mg of the hydroxybenzotriazole ester of 2-(2-aminothiazol-4-yl)oximinoacetic acid. Another 0.24 ml of MSTFA was added along with an additional 0.18 ml of diisopropylethylamine and the solution was allowed to warm to room temperature and stirred for 16 h.

The reaction mixture was then treated with 0.5 ml methanol and concentrated in vacuo and then triturated with ether/hexane to provide 477 mg of a brown solid. Reverse-phase (0-20% $CH_3CN/H_2O$ gradient) liquid chromatography provided 61 mg of the title compound.
Elemental Analysis:
Theory: C, 41.00; H, 3.90; N, 22.31
Found : C, 39.50; H, 3.86, N, 21.40
Mass Spectra (FD): m/e = 440
NMR: 300 MHz ($CDCl_3$): $\delta$ 9.5, bs, 1H; 9.07, d, 1H, J = 9 Hz; 7.17, bs, 2H ($NH_2$); 6.97, s, 1H; 4.94, m, 1H; 4.57, 1H, J = 15 Hz; 4.11, d, 1H, J = 15 Hz; 3.78, s, 3H; 3.66, s, 3H; 3.11, T, 1H, J = 9 Hz.

## Example 13

t-Butyl-7-(S)-(t-butoxycarbonylamino)-3-methylaminocarbonylamino-8-oxo-1,5-diazabicyclo[3.3.0]octa-2-ene-2-carboxylate

Under a nitrogen atmosphere, a 766 mg (2 mM) sample of the 3-carboxy nucleus prepared in Example 171 was dissolved in 8 ml of $CH_2Cl_2$ and treated with 0.47 ml (2.2 mM) of diphenylphosphorylazide followed by 0.24 ml (2.2 mM) of N-methyl morpholine. The reaction mixture was diluted with 80 ml of benzene and refluxed for 3 h. The reaction mixture was allowed to cool to room temperature over a 1 h period and treated with a solution composed of 0.35 ml of diisopropylethylamine (dissolved in 10 ml $CH_2Cl_2$) and 135 mg (2 mM) of methylamine hydrochloride.

The reaction mixture was sitrred for 16 h, concentrated in vacuo and diluted with ethyl acetate. The crude product solution was then washed with 50 ml of saturated $NaHCO_3$ solution, which was, in turn, back-extracted with ethyl acetate. The combined organics were washed with $H_2O$ (1 x 50 ml), 0.2 N HCl (2 x 50 ml), and brine (2 x 50 ml). The organic layer was then dried over anhydrous $Na_2SO_4$, filtered, and concentrated in vacuo to provide 866 mg of a yellow oil. Purification by liquid chromatography (5:1/ethyl acetate/hexane) provided 139 mg (17% yield) of the compound.
Elemental Analysis:

Theory: C, 52.54; H, 7.10; N, 17.02
Found : C, 52.36; H, 6.83; N, 16.95
Mass Spectra (FD): m/e = 411
NMR: 90 MHz ($CDCl_3$): δ 9.04, s, 1H; 5.25, m, 2H; 4.28, d, 1H, J = 14 Hz; 4.8 m, 1H; 4.00, m, 1H; 3.88, d, 1H, J = 14 Hz; 2.8, d, 3H, J = 6 Hz; 2.04, dd, 1H, J = 9 Hz, J = 11 Hz; 1.52, s, 9H; 1.44, s, 9H.
OR: -113.4° at 589 nm (methanol)

## Example 14

t-Butyl-7-(S)-(t-butoxycarbonylamino)-3-methylthiocarbonylamino-8-oxo-1,5-diazabicyclo[3.3.0]octa-2-ene-2-carboxylate

A 766 mg (2 mM) sample of the 3-carboxy nucleus prepared by the method of Preparation 16 was dissolved in 8 ml of $CH_2Cl_2$ and treated with 0.47 ml (2 mM) of diphenylphosphoryl azide followed by 0.24 ml of N-methyl morpholine. The reaction mixture was diluted with 80 ml of benzene and refluxed for about 4 h. The reaction mixture was then cooled to room temperature and treated with an excess of methanethiol and stirred for 16 h.

The reaction mixture was then concentrated in vacuo and redissolved in 100 ml of ethyl acetate. The ethyl acetate solution was then washed sequentially with saturated $NaHCO_3$ solution (2 x 50 ml), $H_2O$ (1 x 50 ml), 0.2N HCl (2 x 50 ml), and brine (2 x 50 ml), dried over anhydrous $Na_2SO_4$, filtered and concentrated in vacuo to provide 2.36 g of a yellow oil. The crude product was purified by liquid chromatography using 95% $CH_2Cl_2$/5% acetone as eluent to provide 95 mg of the title compound.
Mass Spectra (FD): m/e = 428
NMR: 90 MHz ($CDCl_3$: δ 9.56, bs, 1H; 5.08, m, 1H; 5.8, d, 1H; J = 14 Hz, 4.56, m, 1H; 4.01, dd, J = 8 Hz, J = 10 Hz; 3.96, 1H, J = 14 Hz; 2.62, dd, 1H, J = 9 Hz, J = 11 Hz; 2.36, s, 3H; 1.5, s, 9H, 1.4, s, 9H.

## Example 15

t-Butyl-7-(S)-(t-butoxycarbonylamino)-3-chloro-8-oxo-1,5-diazabicyclo[3.3.0]octa-3-ene-2-carboxylate

Under a nitrogen atmosphere, a 0.25 mMol sample of the 3-keto nucleus prepared by the procedure of Example 12 was dissolved in 10 ml of dry $CH_2CH_2$ and cooled in an ice bath. The $CH_2Cl_2$ solution was treated with 0.057 ml (0.325 mM) of diisopropylethylamine followed by 0.044 ml (0.26 mM) of triflic anhydride. After 45 min., the reaction mixture was treated with 76 mg (0.275 mM) of tetrabutyl ammonium chloride and allowed to warm to room temperature. After 1 h, another 76 mg (0.275 mM) of tetrabutyl ammonium chloride was added and the reaction mixture stirred for 3 days.

The reaction mixture was then concentrated in vacuo and redissolved in 15 ml of ethyl acetate. The ethyl acetate solution was washed with $H_2O$ (2 x 10 ml) and brine (2 x 10 ml), dried over anhydrous $MgSO_4$, filtered and concentrated in vacuo to provide 186 mg of crude product. Purification by liquid chromatography using 2:1 hexane/ethyl acetate as eluent provided 10 mg of the title compound.
Mass Spectra (FD): 373 and 390
Elemental Analysis:
Theory: Cl, 9.48
Found : Cl, 9.65
NMR: 90 MHz ($CDCl_3$): δ 5.1, 1, d, 1H, J = 5 Hz; 4.56,m, 1H; 4.12, d, 1H, J = 13 Hz; 4.04, T, 1H, J = 8 Hz; 3.76, d, 1H, J = 13 Hz; 2.7, dd, J = 7 Hz, J = 9 Hz; 1.52, s, 9H; 1.4, s, 9H.

## Example 16

Allyl-7-(R,S)-(t-butoxycarbonylamino)-3-dimethylaminosulfonyl-8-oxo-1,5-diazabicyclo[3.3.0]octa-3-ene-2-carboxylate

A. Preparation of 1,1-diethylphosphonato, dimethylaminosulfonyl-ethene

Under a nitrogen atmosphere, a solution of 9.84 g of dimethylaminomethanesulfonamide in 75 ml of tetrahydrofuran was cooled to -78° C and treated with 50 ml of a 1.6N solution of n-butyl lithium and the resulting mixture stirred for 1 h. The resultant solution was slowly added to a solution of 13.8 g of diethylchlorophosphate dissolved in 50 ml of tetrahydrofuran. The reaction was maintained at -78° C for 2 h and then allowed to warm to room temperature over a 2 h period. The solvent was removed *in vacuo* and the residue triturated with 3 x 75 ml of hexane. The resulting crude product was purified by preparative scale HPLC using a gradient elution of 50/50 toluene/ethyl acetate to 100% ethyl acetate to provide 2.9 g of diethylphosphonato, dimethylamino sulfonyl methane.
Elemental Analysis:
Theory: C, 32.43; H, 7.00; N, 5.40
found : C, 32.18; H, 6.89, N, 5.19
IR ($cm^{-1}$) ($CHCl_3$): 1052, 1160.7, 1258, 1345 NMR: 300 MHz ($CDCl_3$): $\delta$ 4.24, m, 4H; 4.34, d, 2H, J=17 Hz; 3.96, s, 6H; 1.38, T, 3H, J=6 Hz.

A mixture of 0.400 g of paraformaldehyde, 0.094 g of pyrrolidine in a mixture of 20 ml of benzene and 10 ml of glacial acetic acid was refluxed for 20 min. Next, a 2.59 g sample of diethylphosphonato dimethylsulfonylaminomethane was added and refluxing continued for 5 h.

The reaction mixture was then concentrated *in vacuo* and the product determined to contain 70-80% of vinyl phosphonate by NMR. The crude product was purified by liquid chromatography using ethyl acetate as eluent to provide the title compound for Part A.
Elemental Analysis:
Theory: C, 35.42; H, 6.69; N, 5.16
Found : C, 35.20; H, 6.70; N, 5.09
NMR: 300 MHz ($CDCl_3$): $\delta$ 6.76-6.88, m, 2H; 4.22, m, 4H; 3.9, s, 6H (N-$(CH_3)_2$); 1.97, T, 3H, J=6 Hz.

B. Synthesis of 3-dimethylaminosulfonyl nucleus

A 0.43 g sample of 4-(S)-(t-butoxycarbonylamino)-3-oxo-1,2-pyrazolidine was suspended in 25 ml of $CH_2Cl_2$, cooled in an ice bath, and treated with a 0.592 g sample of the vinyl phosphonate prepared in Part A above, dissolved in 5 ml of $CH_2Cl_2$ and stirred at 0° for 2.5 hr. The reaction mixture was then treated with 0.357 g of allyl oxallyl chloride dissolved in 3 ml of $CH_2Cl_2$ followed by 0.765 ml of diisopropylethylamine and stirred at 0° C for 2.5 h. The reaction mixture was then cooled to -20° C and treated with 0.329 ml of DBU and stirred for 20 min. At this time, another 0.329 ml of DBU was added and the reaction allowed to warm to about 0° C over 50 min.

The reaction mixture was concentrated *in vacuo* and redissolved in 75 ml of ethyl acetate. The ethyl acetate solution was washed sequentially with 0.2N HCl (3 x 40 ml), saturated $NaHCO_3$ solution (2 x 40 ml), and brine (2 x 50). The ethyl acetate solution was then dried over anhydrous $Na_2SO_4$, filtered, and concentrated *in vacuo* to provide 1.05 g of crude product. Purification by liquid chromatography (1:1/ethylacetate/hexane) provided 0.442 g of the title compound.
Elemental Analysis:
Theory: C, 47.43; H, 6.09; N, 13.02
Found : C, 47.30; H, 5.83; N, 12.89
Mass Spectra (FD): m/e=430, 431
NMR: 300 MHz ($CDCl_3$): $\delta$ 5.98, m, 1H; 5.38, dd, 2H, J=15 Hz, J=11 Hz; 5.14, d, 1H, J=8 Hz; 4.94, d, 2, J=8 Hz; 4.74, m, 1H; 4.37, d, 1H, J=12 Hz; 4.06, T, 1H, J=9 Hz; 3.95, d, 1H, J=12 Hz; 2.93, m, 1H; 2.9.

## Example 17

Sodium 7(R,S)-[2-(2-aminothiazol-4-yl)-2-(Z)-(methoximinoacetyl)amino]-8-oxo-3-(2-methoximino)methyl-1,5-diazabicyclo[3.3.0]octa-2-ene-2-carboxylate

A. Preparation of 2-(diethylphosphonato)-3-methoximino)-2-propene

A 1.1 g sample of 1-(diethylphosphonato)acetaldehyde was dissolved in 5 ml of methanol containing 0.84 g of granular potassium carbonate. A 0.5 g sample of methoxyamine hydrochloride was added and the reaction mixture refrigerated for 3 days. The methanol layer was decanted, concentrated in vacuo and the residue purified by liquid chromatography (3% methanol/$CH_2Cl_2$) to provide 0.75 g of a yellow oil.

A solution of 1.6 ml of glacial acetic acid, 10 ml of benzene, 0.14 g of paraformaldehyde, and 0.39 ml of pyrrolidine was refluxed for 10 min. The mixture was then cooled to 0°C and treated with 0.75 g of the yellow oil (oxime) prepared above. The reaction mixture was then refluxed for 20 min. utilizing a Dean-Stark trap. Removal of solvent in vacuo provided 1.8 g which was used directly in Part B.

B. (4-t-Butoxycarbonylamino)-1,2-diazolidin-3-one-1-(3′-methoximino-2′-diethylphosphonato-1-yl)

A 0.9 g sample of (4-t-butoxycarbonylamino)-1,2-diazolidine-3-one and the vinyl phosphonate prepared in Part A above was dissolved in 20 ml of methanol and stirred for approximately 16 h under nitrogen. Methanol was removed in vacuo and the residue purified by flash chromatography (10% methanol ($CH_2Cl_2$)) to provide 0.62 g of the title compound.

C. Allyl 7-(R,S)-(t-butoxycarbonylamino-3-methoximinomethyl-8-oxo-1,5-diazabicyclo[3.3.0]octa-2-ene-2-carboxylate

A 0.36 g sample of the compound prepared in Part B above was dissolved in 9 ml of $CH_2Cl_2$ and cooled to 0°C under $N_2$. The reaction mixture was then treated with a 0.13 g sample of allyloxallyl chloride followed by 0.28 g of diisopropylethylamine. The mixture was then allowed to warm to room temperature after 45 min.

The reaction mixture was then cooled to 0°C and treated with 0.2 ml of DBU and stirred at room temperature overnight. Purifidation by liquid chromatography (95% ethyl acetate/5% $CH_2Cl_2$)) provided 60 mg of the bicyclic pyrazolidinone.

NMR: 300 MHz ($CDCl_3$): $\delta$ 1.42 (s, 9H); 2.76 (dd, 1H, J=9, 12 Hz); 3.80 (d, 1H, 15 Hz); 3.91 (s, 3H); 4.10 (t, 1H); 4.38 (d, 1H, J=12 Hz); 4.78 (m, 3H); 5.30 (m, 3H); 5.94 (m, 1H); 8.30 (s, 1H).

MS: $M^+$ = 381

UV: 247 ($\epsilon$=9120); 359 ($\epsilon$=6670)

D. Deprotection, acylation, and deesterification

The "nucleus" prepared in Part C was treated with trifluoroacetic acid, acylated with the hydroxy benztriazole active ester of 2-(2-aminotriazol-4-yl)-2-(Z)-methoximinoacetic acid in the presence of diisopropylethylamine to provide the allyl ester of the title compound.

A 4.5 mg sample of triphenylphosphine, 1.0 mg of palladium acetate, and 15 mg of sodium-2-ethylhexanoate was dissolved in 0.5 ml of acetone. The reaction mixture was then treated with a 40 mg sample of the allyl ester prepared above, dissolved in 1 ml of acetone and stirred for 1 h at room temperature.

The reaction mixture was then centrifuged and the organics decanted. The solids were washed with acetone, centrifuged and again decanted. The combined acetone portions were concentrated in vacuo to provide 40 mg of the title compound.

NMR: 300 MHz ($D_2O$): $\delta$ 3.25 (t, 1H); 3.83-4.12 (m, 2H) with 3.92 (s, 3H) and 3.99 (s, 3H) superimposed; 4.31 (d, 1H); 5.27 (dd, 1H, J=9, 12 Hz); 7.11 (s, 1H); 8.26 (s, 1H).

UV: 234 ($\epsilon$=18,800); 336 ($\epsilon$=12,500)

Example 18

Sodium 7-(R,S)-[2-(2-aminothiazol-4-yl)methoximinoacetyl)amino]-8-oxo-3-benzyloximinomethyl-1,5-diazabicyclo[3.3.0]octa-2-ene-2-carboxylate

In a manner analogous to Example 17 above, the title compound was prepared.
NMR: 300 MHz ($D_2O$): $\delta$ 3.23 (t, 1H); 4.03 (m, 1H) with 3.98 (s, 3H) superimposed; 4.27 (d, 1H); 4.7-4.9 (m, 4); 5.18 (s, 2H); 5.25 (dd, 1H); 7.09 (s, 1H); 7.45 (s, 5H); 8.31 (s, 1H).
IR: 3500 $cm^{-1}$ , 1700, 1630, 1535
Mass Spectra (FD): 456, 455, 108, 93 (100)
UV: 234 ($\epsilon$ = 19,700); 338 ($\epsilon$ = 13,100)

## Claims

1. A compound of Formula (I):

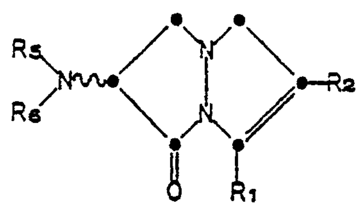

(I)

wherein:
$R_2$ is cyano;
a group of the formula

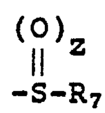

wherein Z is 0, 1 or 2 and $R_7$ is $C_1$ to $C_6$ alkyl, $C_2$ to $C_7$ alkenyl or $C_2$ to $C_7$ alkynyl;
a group of the formula

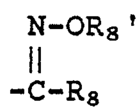

,

wherein $R_8$ is hydrogen, $C_1$ to $C_6$ alkyl, $C_1$ to $C_6$ substituted alkyl, perfluoro $C_2$ to $C_4$ alkyl, trihalomethyl, $C_7$ to $C_{12}$ arylalkyl, $C_7$ to $C_{12}$ substituted arylalkyl, phenyl, substituted phenyl, amino, (monosubstituted)-amino, or (disubstituted)amino; and $R_8'$ is hydrogen, $C_1$ to $C_6$ alkyl, $C_2$ to $C_7$ alkenyl, $C_2$ to $C_7$ alkynyl, phenyl, substituted phenyl, $C_7$ to $C_{12}$ phenylalkyl, $C_1$ to $C_{12}$ substituted phenylalkyl;
or a group of the formula

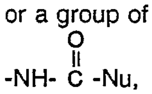

wherein Nu is (monosubstituted)amino, (disubstituted)amino, $C_1$ to $C_6$ alkylthio, $C_2$ to $C_6$ alkenylthio, $C_1$ to $C_6$ substituted alkylthio, phenylalkylthio, $C_7$ to $C_1$ phenylalkylthio, or $C_7$ to $C_{12}$ substituted phenylalkylthio, $C_1$ to $C_6$ alkyl alcohol, $C_1$ to $C_6$ substituted alkyl alcohol, phenyl alcohol, substituted phenyl alcohol, $C_7$ to $C_{12}$ phenylalkyl alcohol, or $C_7$ to $C_{12}$ substituted phenylalkyl alcohol;
and $R_1$ is a group of the formula

$-COOR_{14}$

wherein $R_{14}$ is hydrogen, an organic or inorganic cation, a carboxy-protecting group or a non-toxic, metabolically-labile ester-forming group;
$R_5$ is hydrogen; and

$R_6$ is an acyl group of the formula

$-COR_{16}$

wherein $R_{16}$ is a group of the formula

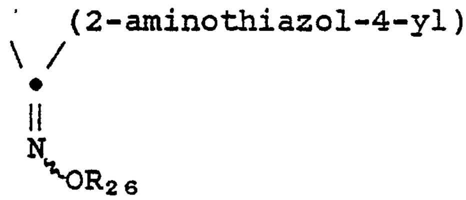

wherein $R_{26}$ is $C_2$ to $C_6$ alkenyl or $C_2$ to $C_7$ alkynyl; or a pharmaceutically-acceptable salt thereof.

2. A compound of claim 1, wherein the 7-$R_5R_6$-N-group is in the (S) configuration.

3. A compound of claim 1 or 2, wherein $R_2$ is cyano.

4. A compound of claim 1 or 2, wherein $R_2$ is a group of the formula

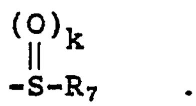

5. A compound of claim 1, 2 or 4, wherein $R_7$ is $C_1$ to $C_6$ alkyl or $C_2$ to $C_7$ alkenyl.

6. A compound of claim 1, 2, 3 or 5, wherein $R_7$ is methyl and z is 2.

7. 7-(S)-[2-(2-Aminothiazol-4-yl)-2-(Z)-(3-butene-1-yl)oximinoacetyl]amino-3-methylsulfonyl-1,5-diazabicyclo[3.3.0]octa-2-ene-2-carboxylic acid or a pharmaceutically-acceptable salt thereof.

8. Any one of the following compounds:

7-(S)-[2-(2-Aminothiazol-4-yl)-2-(Z)-2-propene-1-yl)oximinoacetyl]amino-3-methylsulfonyl-1,5-diazabicyclo-[3.3.0]octa-2-ene-2-carboxylic acid;

7-(S)-[2-(2-Aminothiazol-4-yl)-2-(Z)-4-pentene-1-yl)oximinoacetyl]amino-3-methylsulfonyl-1,5-diazabicyclo-[3.3.0]octa-2-ene-2-carboxylic acid;

7-(S)-[2-(2-Aminothiazol-4-yl)-2-(Z)-2-(allyl)oximinoacetyl]amino-3-methylsulfonyl-1,5-diazabicyclo[3.3.0]octa-2-ene-2-carboxylic acid;

7-(S)-[2-(2-Aminothiazol-4-yl)-2-(Z)-2-propyne-1-yl)oximinoacetyl]amino-3-methylsulfonyl-1,5-diazabicyclo-[3.3.0]octa-2-ene-2-carboxylic acid;

7-(S)-[2-(2-Aminothiazol-4-yl)-2-(Z)-2-(allyl)oximinoacetyl]amino-3-propylsulfonyl-1,5-diazabicyclo[3.3.0]octa-2-ene-2-carboxylic acid;

7-(S)-[2-(2-Aminothiazol-4-yl)-2-(Z)-2-(allyl)oximinoacetyl]amino-3-allylsulfonyl-1,5-diazabicyclo[3.3.0]octa-2-ene-2-carboxylic acid;

or a pharmaceutically-acceptable salt thereof.

9. A pharmaceutical formulation comprising as an active ingredient a compound of Formula (I) as claimed in any one of claims 1 to 8, associated with one or more pharmaceutically-acceptable carriers therefor.

10. A compound of Formula (I) as claimed in any one of claims 1 to 8, for use as an antibacterial.

11. A process for preparing a compound of Formula (I) as claimed in any one of claims 1 to 8, which comprises

a) treatment of a compound of the formula

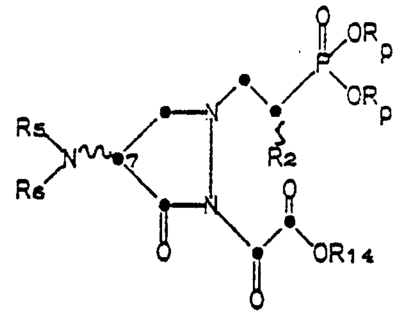

with a suitable base, wherein Rp is $C_1$ to $C_6$ alkyl or phenyl; or

b) treatment of a compound of the formula

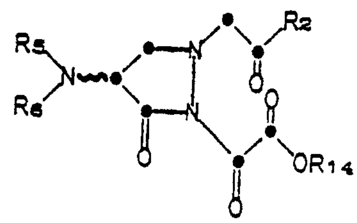

with a phosphite reagent of the formula P $(ORp)_3$, wherein Rp is $C_1$ to $C_6$ alkyl or phenyl; or

c) oxidizing a compound of Formula (I) wherein $R_2$ is

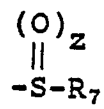

and Z is 0 or 1 to provide a compound of Formula (I) wherein Z is 2;

d) deprotection of carboxy and/or amino groups; or

e) acylation of a compound of the formula

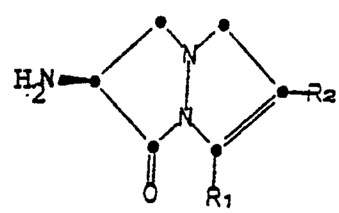

with an activated form of the carboxylic acid

$R_{16}\overset{O}{\overset{\|}{C}}$ -O-H.

12. A process for preparing a compound of Formula (I) wherein $R_2$ is amino, which comprises hydrogenating a compound of the formula

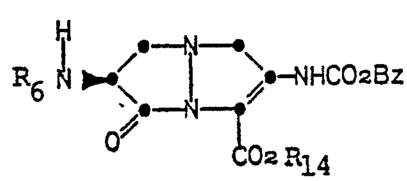

13. A process for preparing a compound of the formula

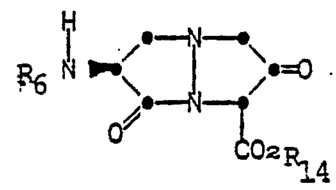

which comprises treating a compound of Formula (I), wherein $R_2$ is amino, with acid.

14. A process for preparing a compound of Formula (I) werehin $R_2$ is

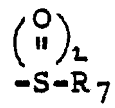

and $R_7$ is (disubstituted)amino, which comprises reacting compounds of the formula

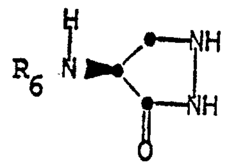

(a)

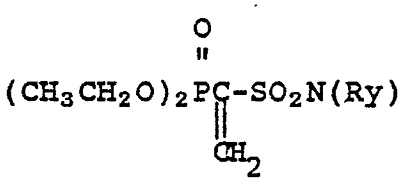

(b)

wherein Ry is phenyl, substituted phenyl, $C_1$ to $C_6$ alkyl, or $C_6$ to $C_{12}$ arylalkyl; and

$Cl-\overset{O}{\overset{\|}{C}}-\overset{O}{\overset{\|}{C}}-O-R_{14}$ (c)

in an inert solvent in the presence of a strong base.

15. A compound of the formula

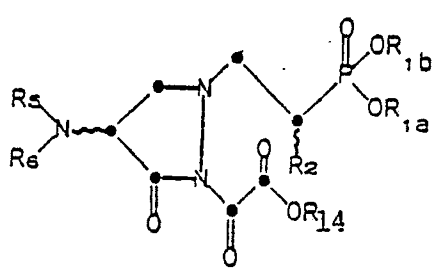

wherein $R_{1a}$ and $R_{1b}$ are the same or different and are $C_1$ to $C_6$ alkyl or phenyl; $R_{14}$ is a carboxy protecting group or a biological-labile ester; and $R_2$ is as defined in Claim 1.

16. A compound of the formula

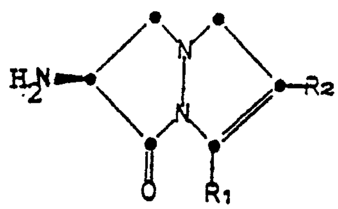

wherein $R_2$ is $C_2$ to $C_6$ akenyl or $C_2$ to $C_6$ alkynyl, or an acid-addition salt thereof.

Claims for the following Contracting State: GR

1. A process for preparing compound of Formula (I):

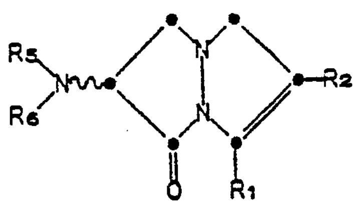

(I)

wherein:
$R_2$ is cyano;
a group of the formula

(O)z
||
-S-$R_7$

wherein Z is 0, 1 or 2 and $R_7$ is $C_1$ to $C_6$ alkyl, $C_2$ to $C_7$ alkenyl or $C_2$ to $C_7$ alkynyl;
a group of the formula

N-$OR_8'$
||
-C-$R_8$ ,

wherein $R_8$ is hydrogen, $C_1$ to $C_6$ alkyl, $C_1$ to $C_6$ substituted alkyl, perfluoro $C_2$ to $C_4$ alkyl, trihalomethyl, $C_7$ to $C_{12}$ arylalkyl, $C_7$ to $C_{12}$ substituted arylalkyl, phenyl, substituted phenyl, amino, (monosubstituted)-amino, or (disubstituted)amino; and $R_8'$ is hydrogen, $C_1$ to $C_6$ alkyl, $C_2$ to $C_7$ alkenyl, $C_2$ to $C_7$ alkynyl, phenyl, substituted phenyl, $C_7$ to $C_{12}$ phenylalkyl, $C_7$ to $C_{12}$ substituted phenylalkyl;
or a group of the formula

O
||
-NH- C -Nu ,

wherein Nu is (monosubstituted)amino, (disubstituted)amino, $C_1$ to $C_6$ alkylthio, $C_2$ to $C_7$ alkenylthio, $C_1$ to $C_6$ substituted alkylthio, phenylalkylthio, $C_7$ to $C_1$ phenylalkylthio, or $C_7$ to $C_{12}$ substituted phenylalkylthio, $C_1$ to $C_6$ alkyl alcohol, $C_1$ to $C_6$ substituted alkyl alcohol, phenyl alcohol,
substituted phenyl alcohol, $C_7$ to $C_{12}$ phenylalkyl alcohol, or $C_7$ to $C_{12}$ substituted phenylalkyl alcohol; and $R_1$ is a group of the formula

$-COOR_{14}$

wherein $R_{14}$ is hydrogen, an organic or inorganic cation, a carboxy-protecting group or a non-toxic, metabolically-labile ester-forming group;
$R_5$ is hydrogen; and

$R_6$ is an acyl group of the formula

$-COR_{16}$ ,

wherein $R_{16}$ is a group of the formula

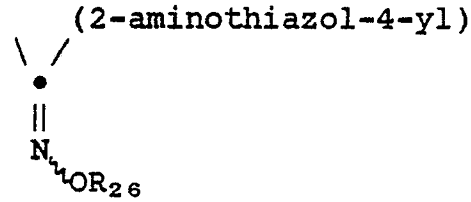

wherein $R_{26}$ is $C_2$ to $C_6$ alkenyl or $C_2$ to $C_7$ alkynyl; or a pharmaceutically-acceptable salt thereof, which comprises:

a) treatment of a compound of the formula

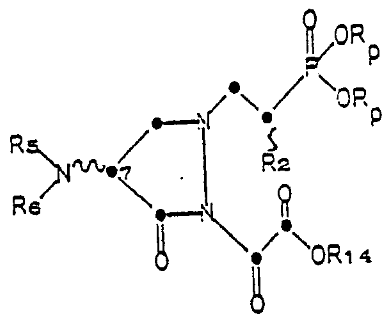

with a suitable base, wherein Rp is $C_1$ to $C_6$ alkyl or phenyl; or

b) treatment of a compound of the formula

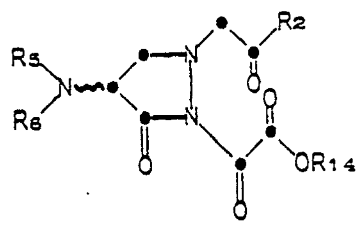

with a phosphite reagent of the formula P $(ORp)_3$, wherein Rp is $C_1$ to $C_6$ alkyl or phenyl; or

c) oxidizing a compound of Formula (I) wherein $R_2$ is

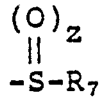

and Z is 0 or 1 to provide a compound of Formula (I) wherein Z is 2 ;

d) deprotection of carboxy and/or amino groups; or

e) acylation of a compound of the formula

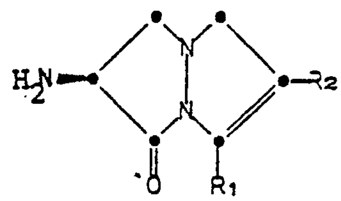

with an activated form of the carboxylic acid

O
$R_{16}$ C -O-H.

2. A process according to claim 1, wherein the 7-$R_5R_6$-N-group is in the (S) configuration.

3. A process according to claim 1 or 2, wherein $R_2$ is cyano.

4. A process according to claim 1 or 2, wherein $R_2$ is a group of the formula

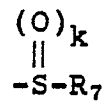

5. A process according to claim 1, 2 or 4, wherein $R_7$ is $C_1$ to $C_6$ alkyl or $C_2$ to $C_7$ alkenyl.

6. A process according to claim 1, 2, 3 or 5, wherein $R_7$ is methyl and z is 2.

7. A process according to any one of claim 1, 2, 3, 5 or 6, wherein the compound of Formula (I) is 7-(S)-[2-(2-Aminothiazol-4-yl)-2-(Z)-(3-butene-1-yl)oximinoacetyl]amino-3-methylsulfonyl-1,5-diazabicyclo-[3.3.0]octa-2-ene-2-carboxylic acid or a pharmaceutically-acceptable salt thereof.

8. A process according to any one of claims 1, 2, 3, 5, or 6, wherein the compound of Formula (I) is any one of the following compounds:
7-(S)-[2-(2-Aminothiazol-4-yl)-2-(Z)-2-propene-1-yl)oximinoacetyl]amino-3-methylsulfonyl-1,5-diazabicyclo-[3.3.0]octa-2-ene-2-carboxylic acid;
7-(S)-[2-(2-Aminothiazol-4-yl)-2-(Z)-4-pentene-1-yl)oximinoacetyl]amino-3-methylsulfonyl-1,5-diazabicyclo-[3.3.0]octa-2-ene-2-carboxylic acid;
7-(S)-[2-(2-Aminothiazol-4-yl)-2-(Z)-2-(allyl)oximinoacetyl]amino-3-methylsulfonyl-1,5-diazabicyclo[3.3.0]octa-2-ene-2-carboxylic acid;
7-(S)-[2-(2-Aminothiazol-4-yl)-2-(Z)-2-propyne1-yl)oximinoacetyl]amino-3-methylsulfonyl-1,5-diazabicyclo-[3.3.0]octa-2-ene-2-carboxylic acid;
7-(S)-2-(2-Aminothiazol-4-yl)-2-(Z)-2-(allyl)oximinoacetyl]amino-3-propylsulfonyl-1,5-diazabicyclo[3.3.0]octa-2-ene-2-carboxylic acid;
7-(S)-[2-(2-Aminothiazol-4-yl)-2-(Z)-2-(allyl)-oximinoacetyl]amino-3-allylsulfonyl-1,5-diazabicyclo[3.3.0]octa-2-ene-2-carboxylic acid;
or a pharmaceutically-acceptable salt thereof.

9. A process for preparing a compound of Formula (I) wherein $R_2$ is amino, which comprises hydrogenating a compound of the formula

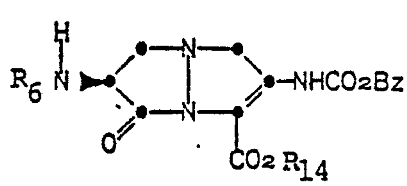

10. A process for preparing a compound of the formula

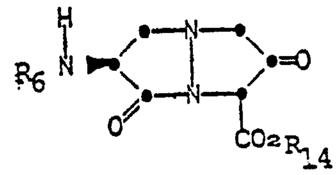

which comprises treating a compound of Formula (I), wherein $R_2$ is amino, with acid.

11. A process for preparing a compound of Formula (I) wherein $R_2$ is

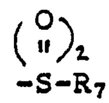

and $R_7$ is (disubstituted)amino, which comprises reacting compounds of the formula

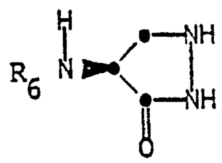

(a)

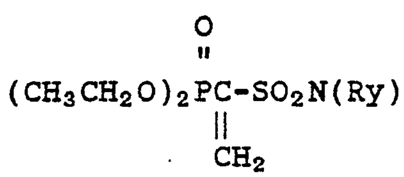

(b)

wherein Ry is phenyl, substituted phenyl, $C_1$ to $C_6$ alkyl, or $C_6$ to $C_{12}$ arylalkyl; and

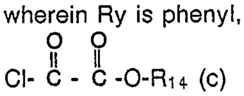 (c)

in an inert solvent in the presence of a strong base.

12. A compound of the formula

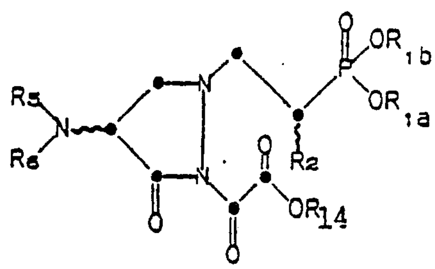

wherein $R_{1a}$ and $R_{1b}$ are the same or different and are $C_1$ to $C_6$ alkyl or phenyl; $R_{14}$ is a carboxy protecting group or a biological-labile ester; and $R_2$ is as defined in Claim 1.

13. A compound of the formula

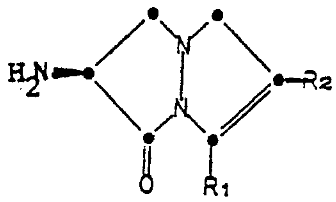

wherein $R_2$ is $C_2$ to $C_6$ akenyl or $C_2$ to $C_6$ alkynyl, or an acid-addition salt thereof.

14. A process for preparing a pharmaceutical formulation which comprises admixing a compound of Formula (I), as defined in any one of claims 1 to 8, or as a pharmaceutically-acceptable salt thereof, with one or more pharmaceutically-acceptable carriers therefor.

Claims for the Following Contracting State: ES

1. A process for preparing compound of Formula (I):

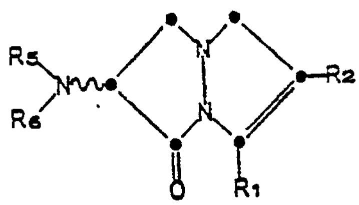

(I)

wherein:
$R_2$ is cyano;
a group of the formula

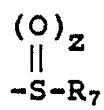

wherein Z is 0, 1 or 2 and $R_7$ is $C_1$ to $C_6$ alkyl, $C_2$ to $C_7$ alkenyl or $C_2$ to $C_7$ alkynyl;
a group of the formula

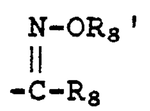

,

wherein $R_8$ is hydrogen, $C_1$ to $C_6$ alkyl, $C_1$ to $C_6$ substituted alkyl, perfluoro $C_2$ to $C_4$ alkyl, trihalomethyl, $C_7$ to $C_{12}$ arylalkyl, $C_7$ to $C_{12}$ substituted arylalkyl, phenyl, substituted phenyl, amino, (monosubstituted)-amino, or (disubstituted)amino; and $R_8'$ is hydrogen, $C_1$ to $C_6$ alkyl, $C_2$ to $C_7$ alkenyl, $C_2$ to $C_7$ alkynyl, phenyl, substituted phenyl, $C_7$ to $C_{12}$ phenylalkyl, $C_7$ to $C_{12}$ substituted phenylalkyl;
or a group of the formula

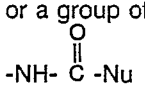

wherein Nu is (monosubstituted)amino, (disubstituted)amino, $C_1$ to $C_6$ alkylthio, $C_2$ to $C_7$ alkenylthio, $C_1$ to $C_6$ substituted alkylthio, phenylalkylthio, $C_7$ to $C_1$ phenylalkylthio, or $C_7$ to $C_{12}$ substituted phenylalkylthio, $C_1$ to $C_6$ alkyl alcohol, $C_1$ to $C_6$ substituted alkyl alcohol, phenyl alcohol, substituted phenyl alcohol, $C_7$ to $C_{12}$ phenylalkyl alcohol, or $C_7$ to $C_{12}$ substituted phenylalkyl alcohol;
and $R_1$ is a group of the formula

$-COOR_{14}$

wherein $R_{14}$ is hydrogen, an organic or inorganic cation, a carboxy-protecting group or a non-toxic, metabolically-labile ester-forming group;
$R_5$ is hydrogen; and
$R_6$ is an acyl group of the formula

$-COR_{16}$ ,

wherein $R_{16}$ is a group of the formula

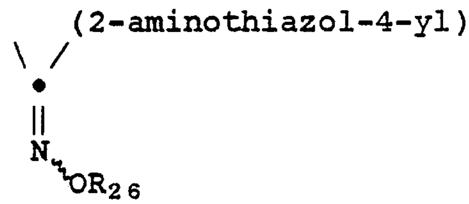

wherein $R_{26}$ is $C_2$ to $C_6$ alkenyl or $C_2$ to $C_7$ alkynyl; or a pharmaceutically-acceptable salt thereof, which comprises:

a) treatment of a compound of the formula

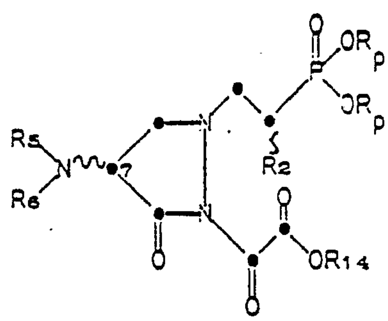

with a suitable base, wherein Rp is $C_1$ to $C_6$ alkyl or phenyl; or

b) treatment of a compound of the formula

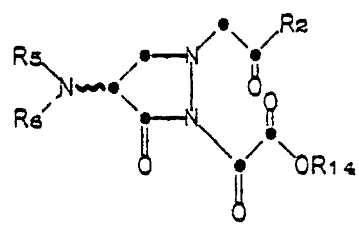

with a phosphite reagent of the formula P $(ORp)_3$, wherein Rp is $C_1$ to $C_6$ alkyl or phenyl; or

c) oxidizing a compound of Formula (I) wherein $R_2$ is

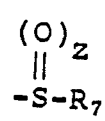

and Z is 0 or 1 to provide a compound of Formula (I) wherein Z is 2;

d) deprotection of carboxy and/or amino groups; or

e) acylation of a compound of the formula

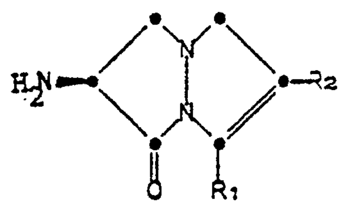

with an activated form of the carboxylic acid

$$R_{16}\overset{O}{\overset{\|}{C}}\text{-O-H}.$$

2. A process according to claim 1, wherein the 7-$R_5R_6$-N-group is in the (S) configuration.

3. A process according to claim 1 or 2, wherein $R_2$ is cyano.

4. A process according to claim 1 or 2, wherein $R_2$ is a group of the formula

$$\overset{(O)_k}{\overset{\|}{-S}}-R_7 \quad .$$

5. A process according to claim 1, 2 or 4, wherein $R_7$ is $C_1$ to $C_6$ alkyl or $C_2$ to $C_7$ alkenyl.

6. A process according to claim 1, 2, 3 or 5, wherein $R_7$ is methyl and z is 2.

7. A process according to any one of claim 1, 2, 3, 5 or 6, wherein the compound of Formula (I) is 7-(S)-[2-(2-Aminothiazol-4-yl)-2-(Z)-(3-butene-1-yl)oximinoacetyl]amino-3-methylsulfonyl-1,5-diazabicyclo-[3.3.0]octa-2-ene-2-carboxylic acid or a pharmaceutically-acceptable salt thereof.

8. A process according to any one of claims 1, 2, 3, 5, or 6, wherein the compound of Formula (I) is any one of the following compounds:
7-(S)-[2-(2-Aminothiazol-4-yl)-2-(Z)-2-propene-1-yl)oximinoacetyl]amino-3-methylsulfonyl-1,5-diazabicyclo-[3.3.0]octa-2-ene-2-carboxylic acid;
7-(S)-[2-(2-Aminothiazol-4-yl)-2-(Z)-4-pentene-1-yl)oximinoacetyl]amino-3-methylsulfonyl-1,5-diazabicyclo-[3.3.0]octa-2-ene-2-carboxylic acid;
7-(S)-[2-(2-Aminothiazol-4-yl)-2-(Z)-2-(allyl)oximinoacetyl]amino-3-methylsulfonyl-1,5-diazabicyclo[3.3.0]octa-2-ene-2-carboxylic acid;
7-(S)-[2-(2-Aminothiazol-4-yl)-2-(Z)-2-propyne-1-yl)oximinoacetyl]amino-3-methylsulfonyl-1,5-diazabicyclo-[3.3.0]octa-2-ene-2-carboxylic acid;
7-(S)-2-(2-Aminothiazol-4-yl)-2-(Z)-2-(allyl)oximinoacetyl]amino-3-propylsulfonyl-1,5-diazabicyclo[3.3.0]octa-2-ene-2-carboxylic acid;
7-(S)-[2-(2-Aminothiazol-4-yl)-2-(Z)-2-(allyl)oximinoacetyl]amino-3-allylsulfonyl-1,5-diazabicyclo[3.3.0]octa-2-ene-2-carboxylic acid;
or a pharmaceutically-acceptable salt thereof.

9. A process for preparing a compound of Formula (I) wherein $R_2$ is amino, which comprises hydrogenating a compound of the formula

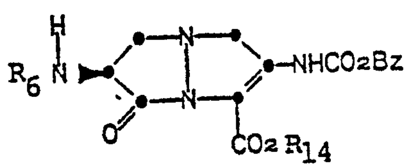

10. A process for preparing a compound of the formula

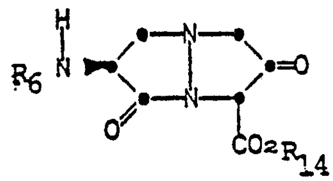

which comprises treating a compound of Formula (I), wherein $R_2$ is amino, with acid.

11. A process for preparing a compound of Formula (I) wherein $R_2$ is

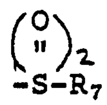

and $R_7$ is (disubstituted)amino, which comprises reacting compounds of the formula

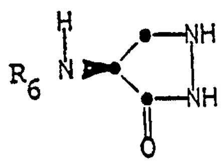

(a)

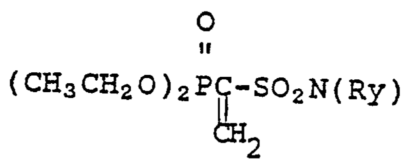

(b)

wherein Ry is phenyl, substituted phenyl, $C_1$ to $C_6$ alkyl, or $C_6$ to $C_{12}$ arylalkyl; and

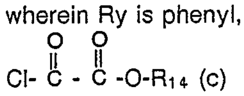
(c)

in an inert solvent in the presence of a strong base.

12. A process for preparing a pharmaceutical formulation which comprises admixing a compound of Formula (I), as defined in any one of claims 1 to 8, or a pharmaceutically-acceptable salt thereof, with one or more pharmaceutically-acceptable carriers therefor.